# EUROPEAN PATENT SPECIFICATION

(11) **EP 2 512 227 B1**
(45) Date of publication and mention of the grant of the patent: **02.11.2016**
(21) Application number: 10788203.7
(22) Date of filing: 03.12.2010
(51) Int. Cl.: A01K 67/027, C12N 15/85

(54) **ANIMAL MODEL EXPRESSING LUCIFERASE UNDER CONTROL OF THE MYELIN BASIC PROTEIN PROMOTER (MBP-LUCI) AND USE OF THE MODEL FOR BIOLUMINESCENCE IN VIVO IMAGING**
TIERMODEL, DAS LUCIFERASE UNTER DER KONTROLLE DES MYELIN BASIC PROTEIN-PROMOTERS EXPRIMIERT (MBP-LUCI) SOWIE VERWENDUNG DES TIERMODELS ZUM BIOLUMINESCENCE IMAGING IN VIVO
MODÈLE ANIMAL EXPRIMANT DE LUCIFÉRASE CONTROLÈE PAR LE PROMOTEUR DE MYELIN BASIC PROTEIN (MBP-LUCI) ET UTILISATION DU MODÉLE POUR BIOLUMINESCENCE IMAGING IN VIVO

(30) Priority: 21.09.2010 FR 1057581; 14.05.2010 US 334637 P; 17.12.2009 US 287371 P
(43) Date of publication of application: 24.10.2012
(60) Divisional application: 16181234.2
(73) Proprietor: SANOFI, 75008 Paris (FR)
(72) Inventor: CAO, James, Bridgewater, New Jersey 08807 (US); CHANDROSS, Karen, Bridgewater, New Jersey 08807 (US); ECONOMIDES, Kyriakos, D., Bridgewater, New Jersey 08807 (US); POLITES, Harry Gregory, Bridgewater, New Jersey 08807 (US); WEINSTOCK, Daniel, Bridgewater, New Jersey 08807 (US); YING, Xiaoyou, Bridgewater, New Jersey 08807 (US)
(74) Representative: Körner, Kathrin
(86) International application number: PCT/US2010/058823
(87) International publication number: WO 2011/084281

(56) References cited:
- WO-A2-2008/022045
- WO-A2-2009/023517
- FARHADI H F ET AL: "A COMBINATORIAL NETWORK OF EVOLUTIONARILY CONSERVED MYELIN BASIC PROTEIN REGULATORY SEQUENCES CONFERS DISTINCT GLIAL-SPECIFIC PHENOTYPES", JOURNAL OF NEUROSCIENCE, NEW YORK, NY, US, vol. 23, no. 32, 12 November 2003 (2003-11-12), pages 10214-10223, XP008037383, ISSN: 0270-6474
- LUO J ET AL: "Bioluminescence in vivo imaging of autoimmune encephalomyelitis predicts disease", JOURNAL OF NEUROINFLAMMATION, vol. 5, 6, February 2008 (2008-02), XP002624068,
- ZENDEDEL A ET AL: 'Cuprizone-Induced Demyelination as a Tool to Study Remyelination and Axonal Protection' JOURNAL OF MOLECULAR NEUROSCIENCE vol. 51, no. 2, 12 October 2013, pages 567 - 572, XP055180981 DOI: 10.1007/s12031-013-0026-4 ISSN: 0895-8696
- CHEN Z ET AL: 'Cuprizone does not induce CNS demyelination in nonhuman primates' ANNALS OF CLINICAL AND TRANSLATIONAL NEUROLOGY vol. 2, no. 2, 18 December 2014, pages 208 - 213, XP055180982 DOI: 10.1002/acn3.159 ISSN: 2328-9503
- BADR C E ET AL: "Bioluminescence imaging: progress and applications", TRENDS IN BIOTECHNOLOGY, vol. 29, no. 12 , pages 624-633, XP028112001, ISSN: 0167-7799, DOI: 10.1016/J.TIBTECH.2011.06.010 [retrieved on 2011-06-20]
- CLOSE D M ET AL: 'In Vivo Bioluminescent Imaging (BLI): Noninvasive Visualization and Interrogation of Biological Processes in Living Animals' SENSORS vol. 11, no. 1, 01 January 2011, pages 180 - 206, XP055180978 DOI: 10.3390/s110100180
- HOCHGRÄFE K ET AL: 'Making the Brain Glow: In Vivo Bioluminescence Imaging to Study Neurodegeneration' MOLECULAR NEUROBIOLOGY vol. 47, no. 3, 01 June 2013, pages 868 - 882, XP055180979 DOI: 10.1007/s12035-012-8379-1 ISSN: 0893-7648

### SEQUENCE LISTING

<110> Cao, James Economides, Kyriakos Polites, Greg Chandross, Karen Weinstock, Daniel
<120> Myelin Basic Protein luciferase (MBP-luci) Bioimaging Model
<130> US2009-166 WO PCT
<160> 8
<170> PatentIn version 3.3
<210> 1
   <211> 24
   <212> DNA
   <213> mouse
<400> 1
   actccttacc acacttcttg cagg 24
<210> 2
   <211> 23
   <212> DNA
   <213> mouse
<400> 2
   tctattgggt gatgtgtgcc atc 23
<210> 3
   <211> 31
   <212> DNA
   <213> mouse
<400> 3
   gggggatcca cctgggacgt agcttttgct g 31
<210> 4
   <211> 29
   <212> DNA
   <213> mouse
<400> 4
   ggggtttaaa ctccggaagc tgctgtggg 29
<210> 5
   <211> 31
   <212> DNA
   <213> mouse
<400> 5
   gggggatcca tccctggatg cctcagaaga g 31
<210> 6
   <211> 29
   <212> DNA
   <213> mouse
<400> 6
   ggggtttaaa ctccggaagc tgctgtggg 29
<210> 7
   <211> 26
   <212> DNA
   <213> firefly
<400> 7
   gaaatgtccg ttcggttggc agaagc 26
<210> 8
   <211> 27
   <212> DNA
   <213> firefly
<400> 8
   ccaaaaccgt gatggaatgg aacaaca 27

## Description

### FIELD OF THE INVENTION

This invention relates to a manufactured Myelin Basic Protein-luciferase (MBP-luci) bioimaging model used, e.g., to visualize and quantify demyelination/remyelination events at transcriptional level in real-time with live animal bioimaging techniques.

### BACKGROUND OF THE INVENTION

In drug development, attrition rates are high with accounts claiming that only one in five compounds makes it through development to approval (DiMasi, JA, et al, J Health Econ 22,151-185). Moreover, despite dramatically increased investment, the rate of introduction of novel drugs has remained relatively constant over the past 30 years, with only two to three advances in new drug classes per year eventually making it to market (Lindsay MA, Nature Rev Drug Discovery, 2, 831-838).

Molecular and functional imaging applied to the initial stages of drug development can provide evidence of biological activity and confirm on-target drug effects. Accordingly, investment in molecular imaging technology is expected to enhance drug development (Rudin M, et al, Progress in Drug Res, 2005, vol 62, 185-255). An advantage of molecular imaging techniques over more conventional readouts is that they can be performed in the intact organism with sufficient spatial and temporal resolution for studying biological processes *in vivo.* Furthermore, these molecular imaging techniques allow for a repetitive, non-invasive, uniform and relatively automated study of the same animal at different time points, thus increasing the statistical power of longitudinal studies and reducing the number of animals required and cost. The MBP-luci model supports both an increase in throughput of early drug candidate screening *in vivo* as well as an increase in biological assay sensitivity. Early lead compounds are often suboptimal as marketed drug products, however, detection of specific and significant activity *in vivo* can lead to the optimization of chemical structures to achieve acceptable levels of activity and minimize toxicity in vivo towards the treatment of specific CNS diseases.

### Molecular Imaging

Molecular imaging refers to the convergence of approaches from various disciplines (e.g., cell and molecular biology, chemistry, medicine, pharmacology, physics, bioinformatics and engineering) to exploit and integrate imaging techniques in the evaluation of specific molecular processes at the cellular and sub-cellular levels in living organism. (Massoud T.F., Genes Dev. 17:545-580)

The advent of genetic engineering has brought about major changes to applied science, including for example the drug discovery pipeline. In the same way, the development and exploitation of animal imaging procedures is providing new means for pre-clinical studies (Maggie A, Ciana P. Nat. Rev. Drug Discvy. 4, 249-255). Animal models traditionally have been cumbersome because of the difficulty in quantifying physiological events in real-time. Over the years new imaging methods have been developed to overcome this difficulty (e.g., MRI, CT, PET). More recently, bioluminescense imaging based on *in vivo* expression of luciferase, the light-emitting enzyme of the firefly, has been used for non-invasive detection of target gene activity.

By combining animal engineering with molecular imaging techniques, it has become possible to conduct dynamic studies on specific molecular processes in living animals. This approach could potentially impact pre-clinical protocols thus widely changing all aspects of medicine (Maggie A. Trends Pharmacolo. Sci. 25, 337).

### Molecular Imaging: Bioluminescent

*In vivo* bioluminescent imaging (BLI) is a sensitive tool that is based on detection of light emission form cells or tissues. The utility of reporter gene technology makes it possible to analyze specific cellular and biological processes in a living animal through *in vivo* imaging methods. Bioluminescence, the enzymatic generation of visible light by a living organism, is a naturally occurring phenomenon in many non-mammalian species (Contag, CH, Mol. Microbiol. 18:593-603). Luciferases are enzymes that catalyze the oxidation of a substrate to release photons of light (Greer LFIII, Luminescence 17:43-74). Bioluminescence from the North American firefly is the most widely applied. The firefly luciferase gene (luc) expression produces the enzyme luciferase which converts the substrate D-luciferin to non-reactive oxyluciferin, resulting in green light emission at 562 nm. Because mammalian tissues do not naturally emit bioluminescence, *in vivo* BLI has considerable appeal because images can be generated with very little background signal.

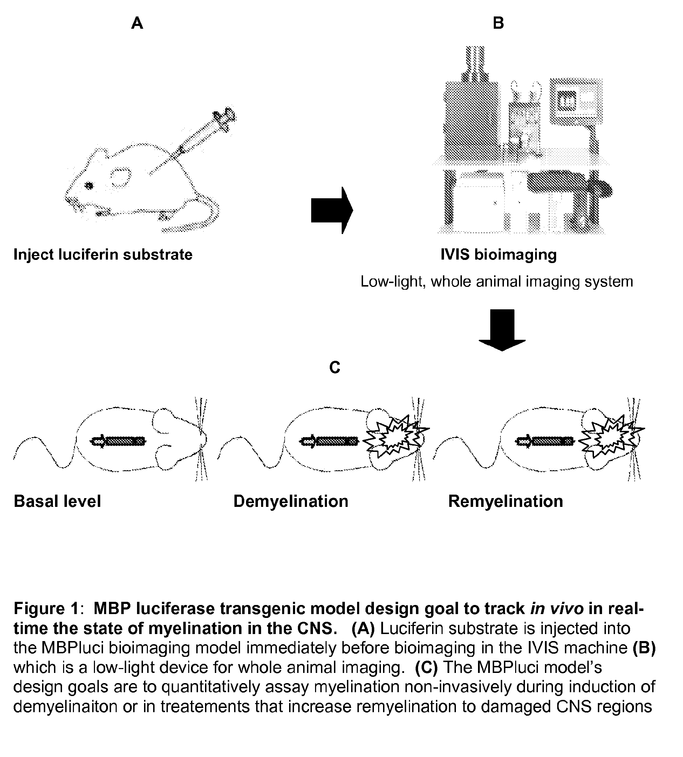

BLI requires genetic engineering of cells or tissues with an expression cassette consisting of the bioluminescent reporter gene under the control of a selected gene promoter driving the light reporter (FIG 1). In order to induce light production, the substrate (e.g., luciferin) is administered by icv, intravascular, intraperitoneal or subcutaneous injection.

The light emitted by luciferase/luciferin is able to penetrate tissue depths of several millimeters to centimeters; however photon intensity decreases about 10 fold for each centimeter of tissue depth (Contag CH Mol. Microbio. 18: 593-603). Sensitive light-detecting instruments must be used to detect bioluminescence *in vivo.* The detectors measure the number of photons emitted per unit area. Low levels of light at wavelengths between 400 and 1000 nm can be detected with charge coupled device cameras that convert the light photons that strike silicon wafers into electrons (Spibey CP et al Electrophoresis 22:829-836). The software is able to convert electron signals into a two-dimensional image. The software is also able to quantify the intensity of the emitted light (number of emitted photons striking the detectors) and convert these numerical values into a pseudocolor graphic. The actual data are measured in photon counts, but the pseudocolor graphic enables rapid visual interpretation. Quantitative measurements within a region of interest may be necessary for more subtle differences. The use of cooled CCD cameras reduces the thermal noise and a light-tight box allows luciferase-produced light to be optimally visualized and quantified (Contag CH, Annu.Rev.Biomed.Eng. 4:235-260).

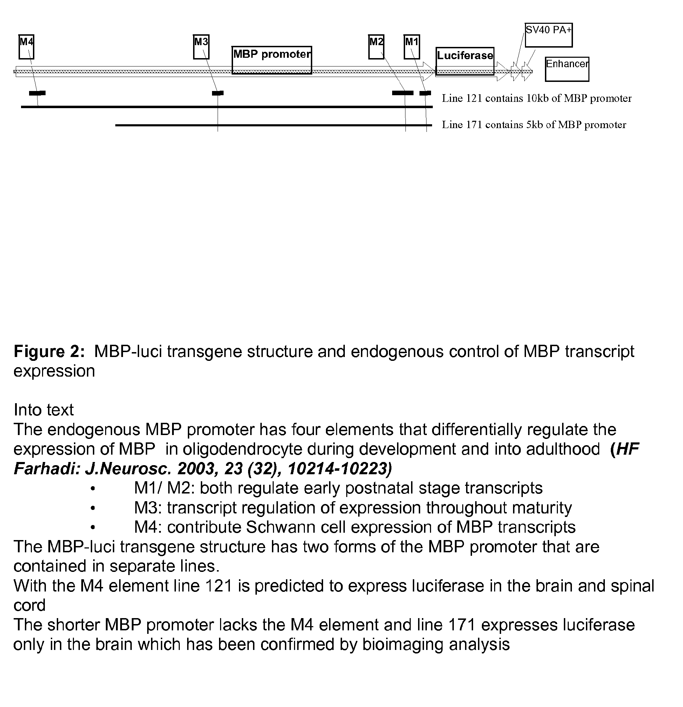

It is useful to have the luciferase image superimposed on another type of image such as autograph or radiograph for anatomical location of the emission signal (Fig 2,). The software superimposes images for visualization and interpretation.

### Demyelinating disease, oligodendrocytes and myelin basic protein

Myelin basic protein (MBP) is required for normal myelin compaction and function. Together with myelin oligodendrocyte glycoprotein (MOG) and proteolipid protein (PLP), these proteins constitute members of the myelin structure protein family and are synthesized by the myelin producing cells: oligodendrocytes in the central nervous system (CNS) and Schwann cells in the peripheral nervous system (PNS). In the developing CNS, the expression of MBP coincides with the time period of myelination. Accordingly, MBP is accepted in the art as a marker for oligodendrocyte maturation. High level expression of MBP (along with other proteins) coincides with myelin elaboration, continues throughout myelinogenesis and ceases upon axon disruption (Gupta et al. Brain Res. 464:133-141)

Diseases that affect myelin integrity result in impaired conduction of axonal signals in the affected neurons, and can result in impaired sensation, movement, cognition, or other functions, depending on which neurons are involved. The term demyelinating diseases describes the effect of the disease, rather than its cause, and can be caused by genetics, infectious agents, autoimmune reactions, and unknown factors. All these remain as human conditions with a huge unmet medical need often associated with the need for restorative therapies.

Demyelinating diseases of the central nervous system include:
- Multiple Sclerosis (together with the similar diseases called idiopathic inflammatory demyeiinating diseases)
- Transverse Myelitis
- Devic's disease
- Progressive Multifocal Leukoencephalopathy
- Optic neuritis
- Leukodystrophies
- Pelizaeus-Merzbacher disease
- Myelin dysfunction / loss has also been associated with Spinal Cord Injury, Alzheimer's and Parkinson's disease, and Schizophrenia

Demyelinating diseases of the peripheral nervous system include:
- Guillain-Barré syndrome and its chronic counterpart, chronic inflammatory demyelinating polyneuropathy
- Peripheral neuropathies (toxin induced, diabetic, anti-MAG etc.).
- Charcot-Marie-Tooth Disease

### In vivo models of demyelination

A common step in the development of pre-clinical candidates to combat demyelinating diseases is assaying the action of therapeutic candidates in animal models that recapitulate part or all of the action of demyelination and which has the capacity for endogenous remyelination. Chemically induced demyelination can be achieved in animal models and young adult male mice at 6-8 weeks old are susceptible to demyelination produced by a 4-6 week diet of 0.2% cuprizone (bis-cyclohexanone oxaldihydrazone) (Ludwin SK , 1978, Lab Invest 39: 597-612). With cuprizone treatment, demyelination occurs globally throughout the brain, but is most easily detected within the highly concentrated white matter region, the corpus callosum (Merkler et al, 2005, NMR Biomed 18: 395-403). Demyelination is evident within 3 weeks after starting the cuprizone diet. Replacement of the diet with normal food allows for almost complete remyelination within 4-6 weeks (Matsushima et al, 2001, Brain Pathol 11: 107-116). Immunohistochemical staining for axonal damage using amyloid precursor protein or Bielschowsky' silver impregnation does not reveal much axonal damage in 8-weekd old mice (Merkler). However, axonal transections increase significantly in 6-7 month old mice, contributing in part to the decline in remyelination seen in aged animals (Irvine KA, et al, 2006, J Neuroimmunol 175: 69-76). Oligodendrocyte progenitor cells, which upon differentiation, replace the myelin, and both microglia and macrophage peak at around 4 weeks on the cuprizone diet and are required for efficient remyelination (Franco RJM, 2002, Nat Rev 3: 705-714).

To quantify changes in myelin content, the entire brain, or subregions, can be harvested and assessed by dot blot or Western analysis- Alternatively, a higher resolution acute (versus longitudinal) approach to track subregions of myelin loss and repair involves quantitative stereology. For example, the computer assisted stereological toolbox (CAST) system can be used to assess myelin Luxol Fast Blue (myelin stain) changes in the corpus callosum. Toluidine blue and Electro Microscopy are necessary final steps to assure that compact myelin is properly ensheathing the previously naked axons. In terms of longitudinal assessment, T2 weighted Magnetic Resonance Imaging provides a means to quantitatively assess myelin changes in the corpus callosum of cuprizone-fed mice (sanofi-aventis Neurology, 2008 unpublished results).

### MBP-luci bioimaging model that tracks myelination status in vivo in real-time

In a 2003 paper, Farhadi et al, (The Journal of Neuroscience, November 12, 2003, 23(32):10214 -10223) reported that the MBP promoter contains regulatory elements, four widely spaced conserved modules, M1, M2, M3 and M4, ranging from 0.1 to 0.4 kb that are critical for regulating the timing of myelination and remyelination (See Fig 1, 3, and 4). Using Lac Z as a reporter gene, they demonstrated that the proximal modules M1 and M2 drive relatively low-level expression in oligodendrocytes during CNS development, whereas the upstream, M3 region, drives high-level expression in oligodendrocytes throughout development and in the adult CNS. Moreover, this M3 region is required for expression during remyelination after a demyelinating insult. M4 also drives MBP expression in myelinating Schwann cells.

Current methods using animal models to assay changes in myelin *in vivo* are time-consuming, typically running between 4 to 8 weeks, and require a large number of animals. The MBP DNA promoter that includes all 4 specific regulatory regions expressing a luciferase gene constitutes the basis for a murine MBP-luci transgenic bioimaging model that was developed to track changes in myelin basic protein (MBP) transcriptional activity in the brain, spinal cord, and/or peripheral nervous system, in real time. This rodent transgenic model can be used for *in vitro* and *in vivo* proof of principle studies intended to select development candidates for the treatment of human demyelinating diseases and offer multiple improvements over the current models:
o The ability to non-invasively track changes in nervous system myelination by bioluminescence reduces the significant resource and manpower demands associated with post-mortem histochemical and tissue expression analyses.
∘ Longitudinal studies greatly increase the sensitivity of the model to changes in myelination. Previous assays have to use separate groups of animals for each time point and this limits the number of data points that can be collected during a study. Additional variables are introduced because of the requirement for separate groups of animals.
∘ Bioimaging data are processed rapidly, typically in the same day, thereby allowing adjustments (i.e. extending the study length to reach significance or early termination due to lack of drug impact) to optimize the study design and reduce unnecessary resource investments.
∘ Animals groups and corresponding treatments can all be assayed at time zero, which optimizes the statistical significance of the study while simultaneously reducing the number of animals needed per treatement group. Study data can be normalized to initial values, greatly reducing inherent biological variation while supporting stronger conclusions on the effects of specific therapies.
∘

### Summary of the Invention

The scope of the present invention is defined in the appended claims.

The present invention provided a new *in vivo* model to assess the effects of therapeutic entities on the extent of demyelination (e.g., neuroprotection, myelin protection) and remyelination (e.g., repair). The bioimaging animals and methods of the present invention allow the screening and/or proof of principle validation of pharmaceutical compounds and other therapeutic agents via the generation of relevant real-time high resolution data in living animals. Additionally, assessments can be obtained relevant to potential toxicity, PK/PD, and the therapeutic window, thereby allowing more accurate predictions around dosing in primates and humans.

The present invention provides improved tools and methods for studying myelination events using a living transgenic rodent. The invention decreases the overall number of subjects required compared to other models, since each subject can serve as its own baseline control in longitudinal studies. Inter-organism variability is thus decreased improving the confidence in statistical results. In various aspects of the invention different advantages are achieved. Several of the various uses are characterized in the following discussion.

By correlation with MBP expression, the present invention provides methods of monitoring demyelination and/or remyelination events in a living rodent. The method comprises transgenically modifying progenitor cells to produce a rodent that expresses a luciferase gene driven through a MBP promoter. The luciferase gene, when expressed can function to produce light (bioluminescence) in the presence of a substrate such as luciferin. By monitoring bioluminescence from the organism myelination and demyelination activities are assessed. Imaging apparatus and analysis rapidly allows for correlating bioluminescence and myelination events to specific portions of the body of the rodent.

For studying events of the central and peripheral nervous systems, vertebrate organisms that employ myelin in these nervous tissues are especially suitable model organisms. Mammals are organisms that use myelin to aid neuronal conduction both in the CNS and PNS. According to the present invention, common mammalian models, namely rodents (e.g., rat, mouse) are the model organisms for use in practicing the present invention.

The organism size is not *per se* a limiting factor. However, apparatus size may be optimized to a particular organism shape or size.

A single bioimaging event may provide desired information. However, an advantage of the present invention provides the ability to repeat measurement on the same animal over a plurality of time points and comparing the multiple measurements. A single organism thereby serves as its own control or baseline.

Consequently, bioimaging over one or more demyelination or remyelination intervals in a single organism is possible. Moreover, imaging signal normalization through demyelination or remyelination intervals, wherein imaging through repeated intervals is effective to detect changes in the level of MBP transcript in an organism over one or more events produces more robust data.

Bioluminescent signal is attenuated by body tissue. Accordingly, nervous tissue proximal to a body surface produces a stronger signal. Signal at the detector can be increased by producing higher signal output by reacting more luciferase, e.g., using higher concentration or increasing enzyme activity or expression levels. Data collection can also be improved using higher sensitivity detectors. Higher sensitivity detectors often require cooling apparatus to produce significant signal and to minimize noise. Time over which data are collected also serves to increase the amount of signal.

The model organism of the present invention is a transgenic rodent comprising a luciferase gene that is driven through a MBP promoter. Rat and mouse are common rodent models. The luciferase gene under control of MBP promoter is expressed in specific target tissues of the model when the promoter is activated or turned on.

The MBP promoter contains M1 through M3 or contains M1 through M4.

Signal improvement can be effected by simple manipulations such as selection of a model whose hair contributes less attenuation. For example, a model whose hair is less attenuating than hair of a C57/B6 mouse will provide superior signal to that of the C57/B6 mouse.

The present invention also provides a method for developing a bioimaging mouse strain in accordance with claim 8.

A cuprizone demyelination model is appropriate for use with the present invention. Timing of bioimaging will depend on the development characteristics of the model organism, for example, one may select timing to take into account that peak of post natal myelination commonly is approximately 3-5 weeks G1 mice.

The present invention is useful for screening chemical compounds, biologics, and other therapeutic entities that may affect myelin events in an organism. A compound may modulate gene expression or intracellular or intercellular signaling events to affect myelin events. These are just some specific examples and are not to be considered as the full scope of the invention which is characterized in the claims.

Although MBP promoter has been well characterized using reporter lac Z gene as described, e.g., Farhadi, above, the use of MBP-lac Z models is limited due to tissue harvest and fixation requirement for detection of β-galactosidase (the *lacZ* report gene product). This process requires histochemical techniques not compatible with detection in living animals. To circumvent this problem, the use of bioluminescence or fluorescence systems has been suggested to develop transgenic bioimaging model in which the luciferase or GFP reporter is selectively controlled by large portions of the MBP promoter.

This novel bioimaging model is designed for the visualization and quantification of demyelination and/or remyelination events in living rodents, e.g., mice, in real-time. Such monitoring can be used in conjunction with automated bioimaging techniques. The model is a useful tool for, e.g., target validation (for example when bioimaging model mice are crossed to knockout and transgenic mice having desired traits), and also for compound validation (e.g., measuring efficacy of a compound in a model such as cuprizone or experimental autoimmune encephalomyelitis [EAE]) on a comparative and quantitative basis to make critical path decisions regarding target selection and compound progression.

The present bioimaging model (MBP-luci TG) has been developed and used for quantifying demyelination/remyelination events *in vivo.* An advantage of the bioimaging model is that a longitudinal study is enabled so that each organism can serve as its own control. Sacrifice of animals at specific time points is thus avoided. Individual mice can be tracked through the demyelination and remyelination process continuously.

The bioimaging methodology requires less time and resources to track biological response in live mice.

### Brief Description of Pictures

Figure 1 shows a schematic summary of MBP luciferase transgenic model, which is used to track real-time changes in myelin expression.
Figure 2 shows that the endogenous MBP promoter has four elements that differentially regulate the expression of MBP in oligodendrocytes during development and into adulthood. ***(***HF Farhadi: J.Neurosc. 2003, 23 (32), 10214-10223***).*** M1/ M2 both regulate early postnatal stage transcripts. M3 is involved in transcript regulation of expression throughout maturity. M4 contributes Schwann cell expression of MBP transcripts. The MBP-luci transgene structure has two forms of the MBP promoter. Line 121 contains the M4 element and is predicted to express luciferase in the brain, spinal cord, and peripheral nervous system. Line 171 consists of the shorter MBP promoter (e.g., lacks the M4 element) and expresses luciferase mainly in the brain, as confirmed by bioimaging analysis.

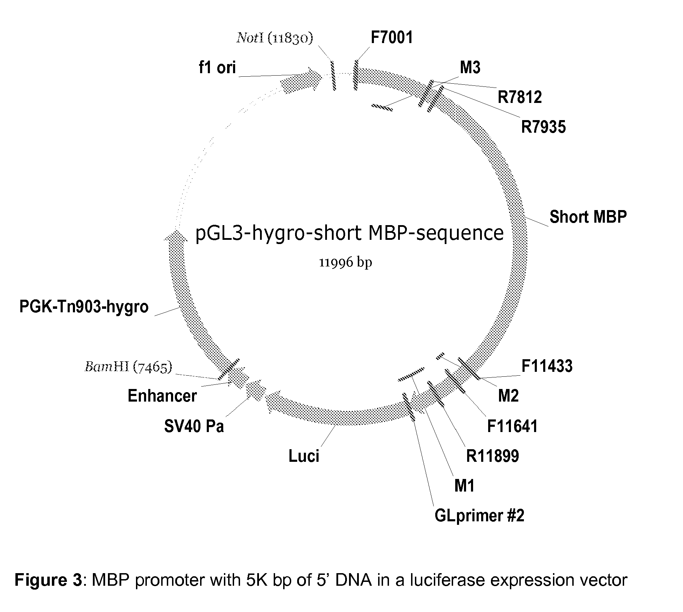

Figure 3 shows an MBP-luci transgene expression cassette including MBP promoter with 5K bp of 5' DNA in a luciferase expression vector.

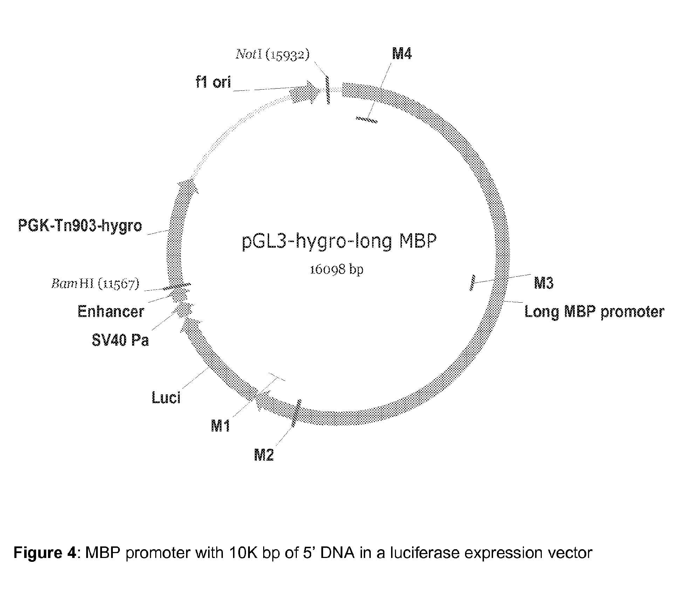

Figure 4 shows an MBP-luci transgene expression cassette including MBP promoter with 10K bp of 5' DNA in a luciferase expression vector.

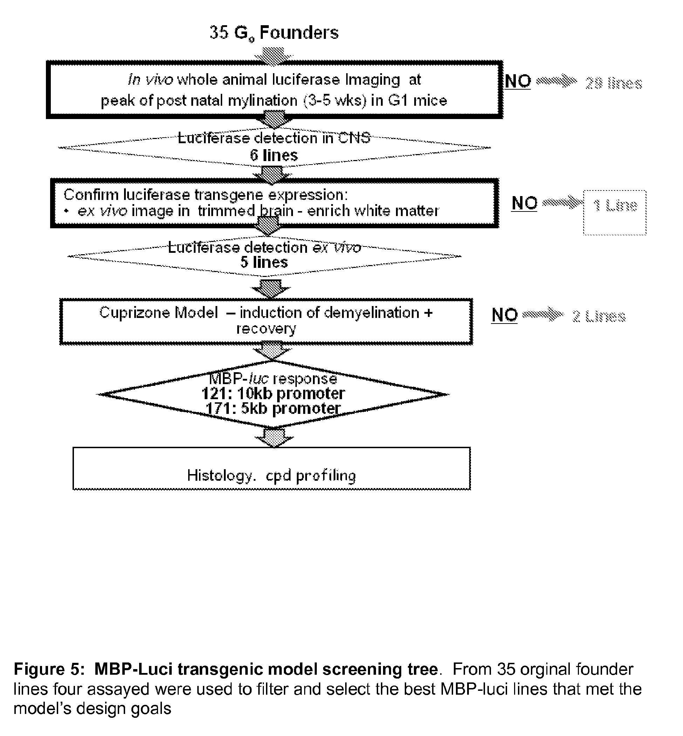

Figure 5 shows an example of the MBP-Luci transgene line screening tree.

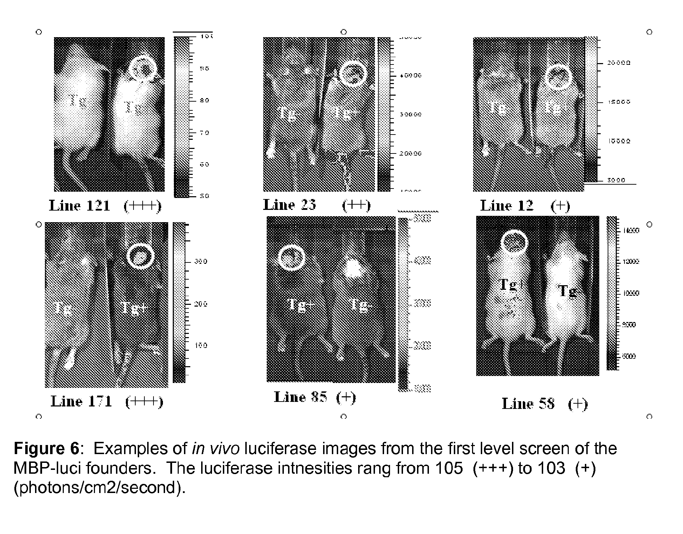

Figure 6 shows *in vivo* luciferase images from the first level screen of the MBP-luci founders. The luciferase intensities rang from 10⁵ (+++) to 10³ (+) (photons/cm2/second).

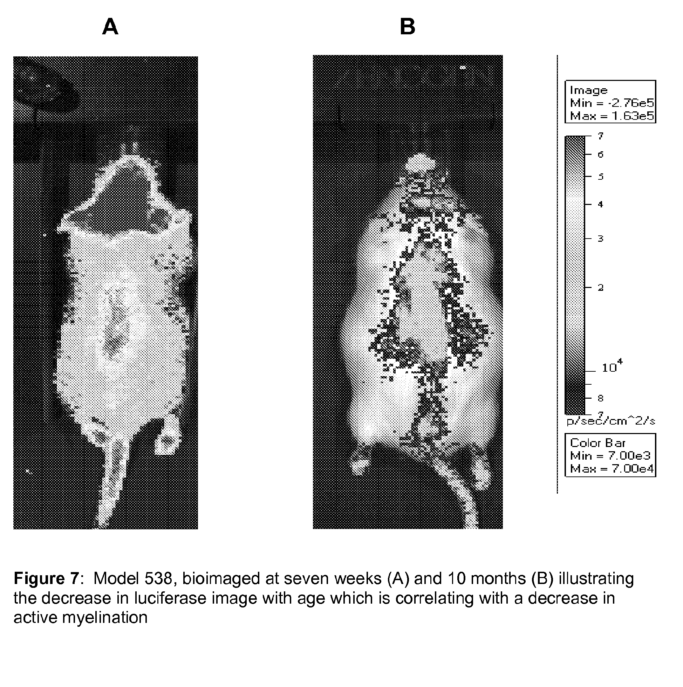

Figure 7 shows a model organism bioimaged at seven weeks (A) and 10 months (B) illustrating the decrease in luciferase image in the brain with increasing age, which correlates with the decrease in myelination observed after early postnatal development.

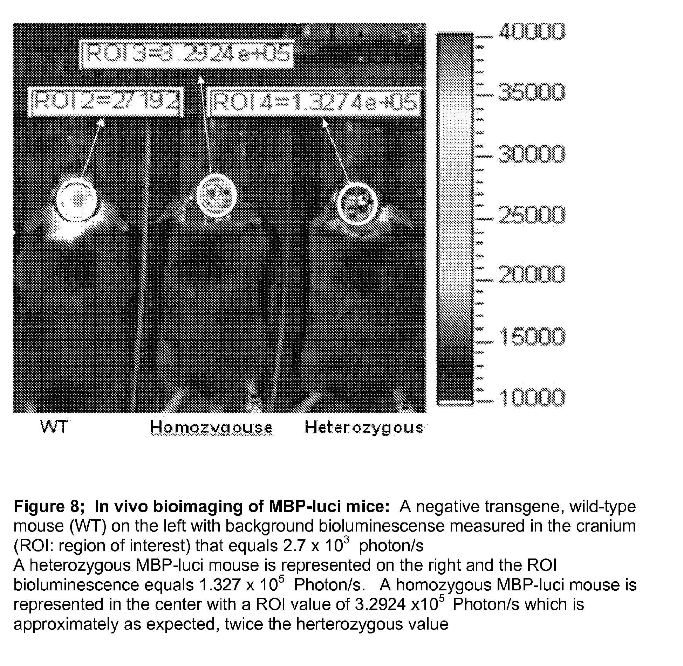

Figure 8 shows *in vivo* bioimaging of MBP-luci mice: A negative transgene, wild-type mouse (WT) on the left with background bioluminescence measured in the cranium (ROI: region of interest) that equals 2.7 x 10³ photon/s A heterozygous MBP-luci mouse is represented on the right and the ROI bioluminescence equals 1.327 x 10⁵ Photon/s. A homozygous MBP-luci mouse is represented in the center with a ROI value of 3.2924x10⁵ Photon/s which is approximately as expected, twice the heterozygous value.

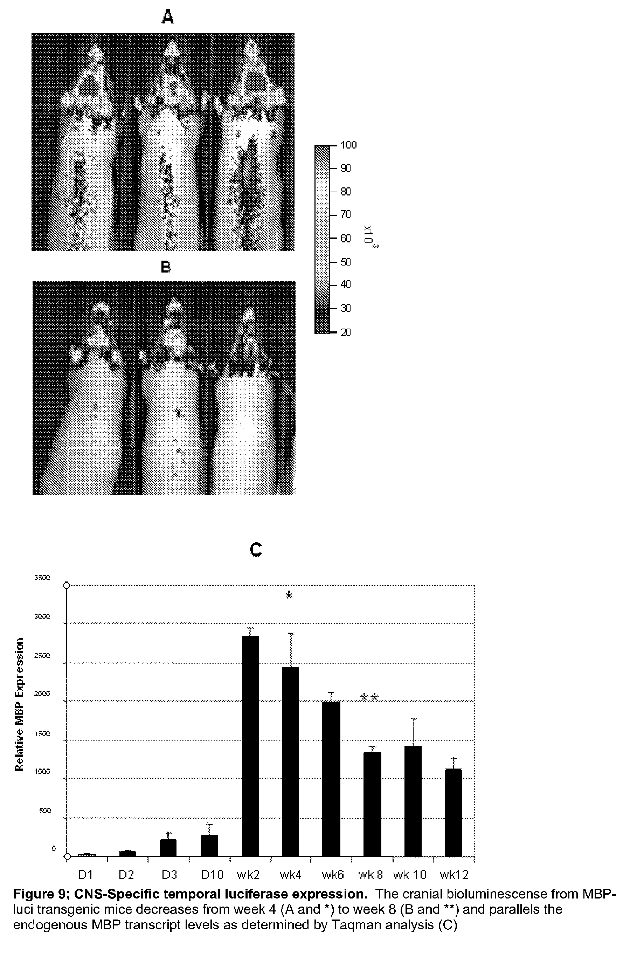

Figure 9 shows CNS-specific temporal luciferase expression. The cranial bioluminescence from MBP-luci transgenic mice as shown decreases from week 4 (A and *) to week 8 (B and **) and parallels the endogenous MBP transcript levels, as determined by Taqman analysis (C).

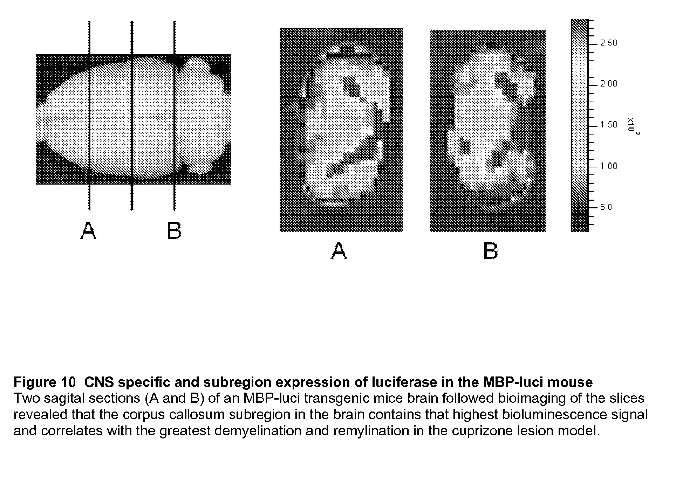

Figure 10 shows CNS specific and subregion expression of luciferase in the MBP-luci mouse. Two sagital sections (A and B) of an MBP-luci transgenic mice brain followed bioimaging of the slicers revealed that the corpus callosum subregion in the brain contains that highest bioluminescence signal and correlates with the greatest demyelination and remyelination in the cuprizone lesion model.

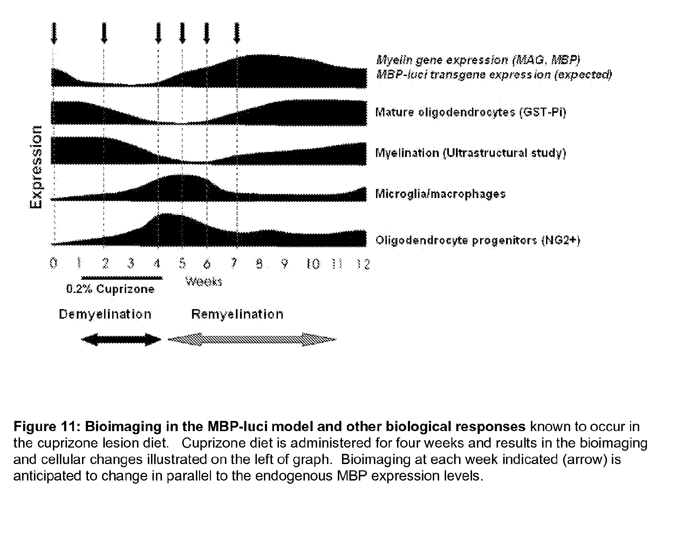

Figure 11 shows Bioimaging in the MBP-luci model and other biological responses known to occur in the cuprizone lesion diet. Here cuprizone diet is administered for four weeks and results in the bioimaging and cellular changes illustrated on the left of graph. Bioimaging at each week indicated (arrow) is anticipated to change in parallel to the endogenous MBP expression levels **(**Matsushima GK and Morell P, Brain Pathology 11, 1-10, 2001**).**

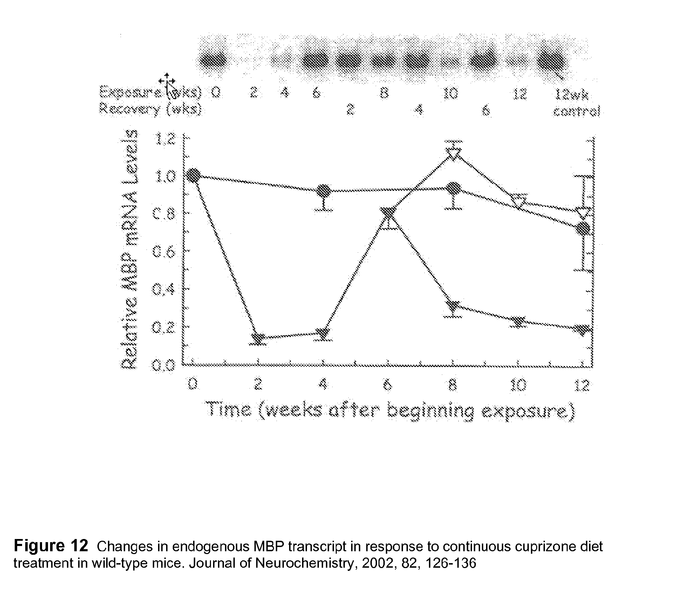

Figure 12 shows changes in endogenous MBP steady-state mRNA in response to continuous cuprizone diet treatment in wild-type C57 BL/6J mice. Mice at 8 weeks of age were exposed to cuprizone in their diet for up to 12 weeks (solid triangle). In second group (open triangle), cuprizone food was removed after 6 weeks of exposure and mice allowed to recover for up to 6 additional weeks. The data for mRNA levels are single determinations by northern blot and are plotted relative to the mean of three controls **(**Jurevics H, et al, Journal of Neurochemistry, 2002, 82, 126-136**).**

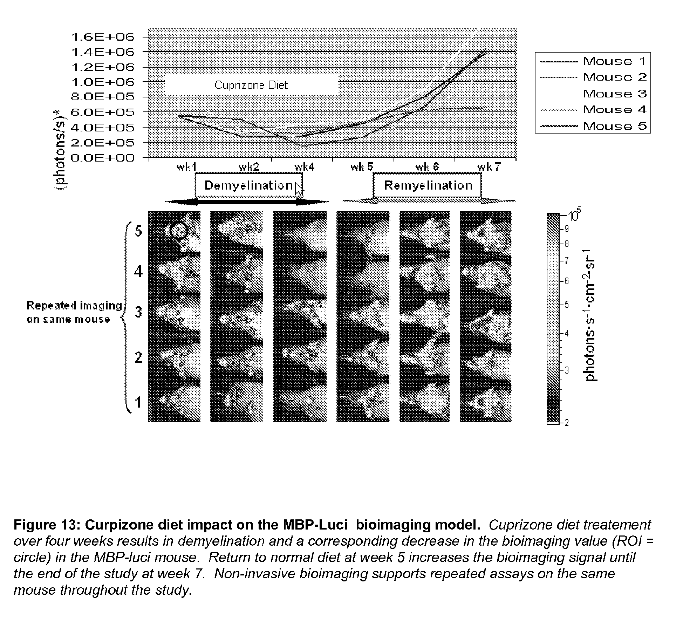

Figure 13 shows a demonstrated cuprizone diet impact on the MBP-Luci bioimaging model. The imaging signal can clearly mimic the expression pattern of endogenous MBP gene. There is a two to three fold imaging signal decrease during cuprizone food feeding (from week 0 to week 4) and also a three to four fold imaging signal increase following removal of cuprizone food (from week 4 to week 7).

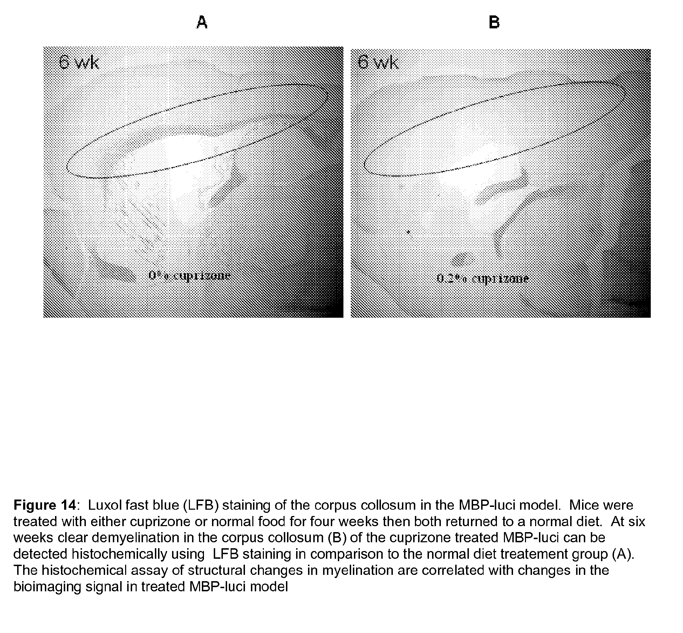

Figure 14 shows luxol fast blue (LFB) staining of the corpus collosum in a MBP-luci model. Mice were treated with either cuprizone or normal food for four weeks then both returned to a normal diet. At six weeks clear demyelination in the corpus collosum (B) of the cuprizone treated MBP-luci can be detected histochemically using LFB staining in comparison to the normal diet treatment group (A). The histochemical assay of structural changes in myelination are correlated with changes in the bioimaging signal in treated MBP-luci model.

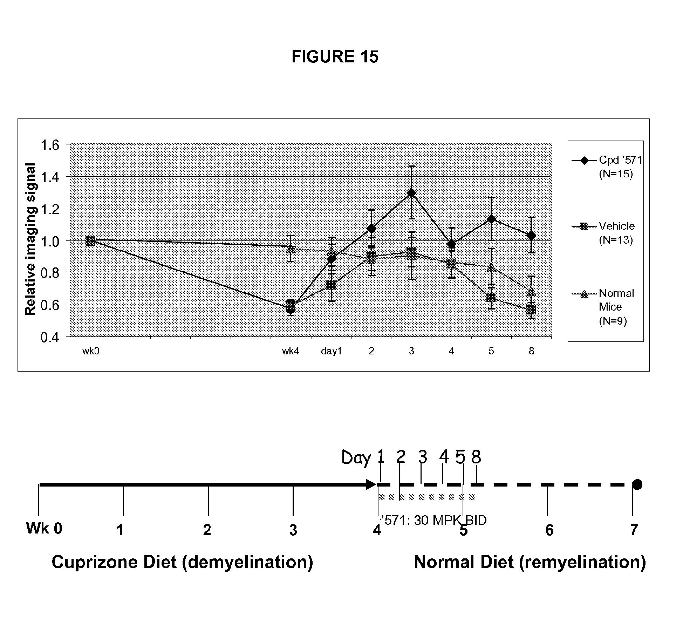

**Figure 15** shows that (hereinafter '517), a Peroxisome Proliferator Activated Receptor delta (PPARδ) agonist tool compound, enhances the imaging signal of luciferase in line 171 het mice during the period of spontaneous remyelination. Eight week- old MBP-luci mice (line 171 heterozygous, B6C3H strain) were placed on a diet containing 0.2% Cuprizone for 4 weeks, then put on a normal chow diet to allow for remyelination. Mice were then dosed orally twice daily with either vehicle (0.6% Carboxymethylcellulose sodium salt and 0.5% Tween 80) or 30 mg/kg PPARδ agonist tool compound '517 for 8 days and imaged at the indicated time points. Data were normalized to week 0 baseline signals. The tool compound, '517 (n=12), caused a 30-100% relative increase in luciferase signal compared to the vehicle group (n=12), which has been attributed to the effect of the compound on stimulating oligodendrocyte progenitor cell differentiation (e.g., consistent with *in vitro* findings).

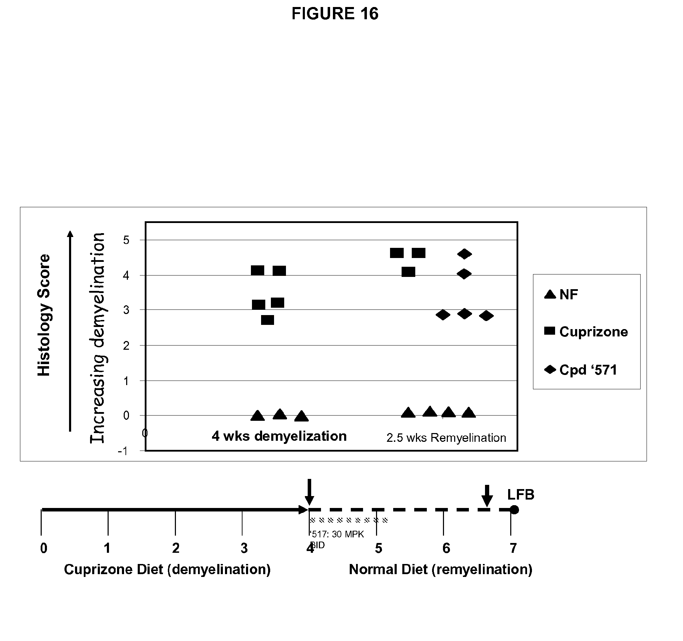

**Figure 16****:** PPARδ agonist tool compound, '517, improves Luxol Fast Blue (LFB) myelin staining during the remyelination phase (line 171 heterozygous, B6C3H strain). Parasagittal tissue sections from formalin fixed paraffin embedded brain were stained with LFB for qualitative assessment of myelin in the corpus callosum. The stained sections for each time point were scored and graded on a scale from 0 (complete myelination) to 5 (complete demyelination). Scoring system was as follows: 0 = normal myelin, no demyelination, 1 = minimal, localized demyelination, 2 = mild to moderate, localized demyelination, 3 = moderate, locally extensive demyelination, 4 = severe, locally extensive demyelination, 5 = severe, diffuse demyelination. Histological evaluation of LFB-stained brain sections from mice, after 4 weeks on cuprizone, confirmed moderate to severe demyelination of the corpus callosum in line 171 heterozygous mice (n=5). Treatment with v from week 4 to week 5 during remyelination phase resulted in a measurable increase in myelin as determined by LFB at the 7 week time point, tool compound '517 group (n=5) compared to vehicle control group (n=3). Despite small n-numbers, histological data support the *in vivo* luciferase bioimaging data, indicating increased remyelination in mice treated with the '517 compound when compared to vehicle controls.

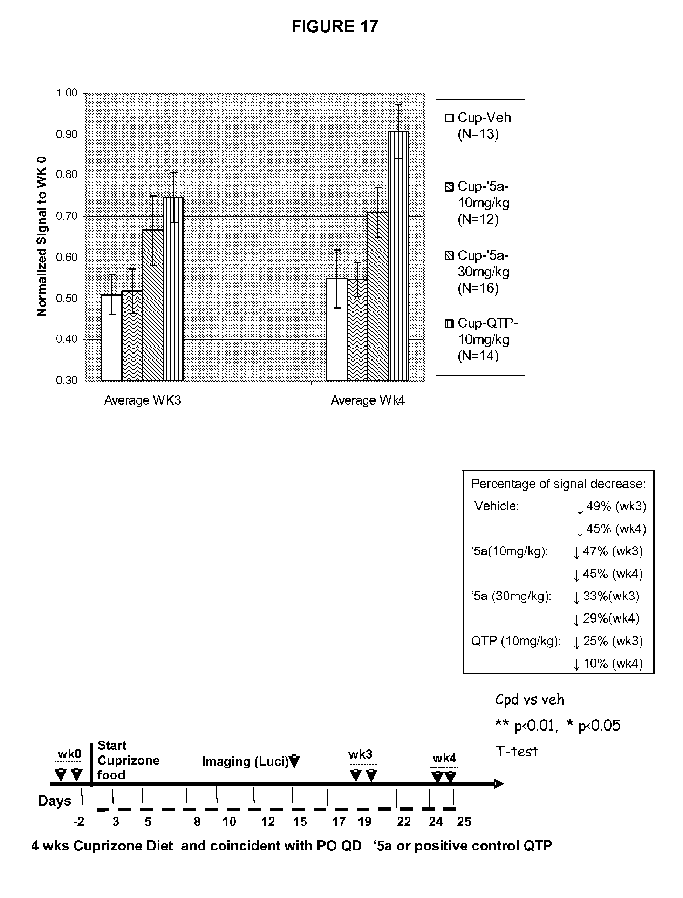

**Figure 17****:** An Estrogen Receptor beta (ERβ) agonist (hereinafter '5a) at 30mg/kg and a positive control Quetiapine at 10mg/kg are protective during the demyelination phase in a cuprizone model. MBP-luc line 171 heterozygous B6C3H mice were fed a diet of cuprizone for 4 weeks and dosed orally with '5a (10mg/kg or 30mg/kg) or the positive control Quetiapine (QTP). Mice were imaged at week 0 (baseline), week 3 and week 4 data normalized to the week 0 baseline. The QTP group showed significant increases in imaging signal vs vehicle control for both week 3 and week 4 time points at 10mg/kg. Results for the QTP treatment group are consistent with data published by Yanbo Zhang et. al., titled "Quetiapine alleviates the cuprizone-induced white matter pathology in brain of C57BL/6 mouse" (Schizophrenia Research, 2008, Dec 106, 182-91). Compound '5a at 30mg/kg showed an enhanced signal compared to vehicle at 3 (trend) and 4 (significant) weeks on the cuprizone diet. '5a at 10 mg/kg had no significant effect at both week 3 and week4. Results suggest that the MBP-luci imaging model is sensitive enough to detect dose dependent changes in the cuprizone model.

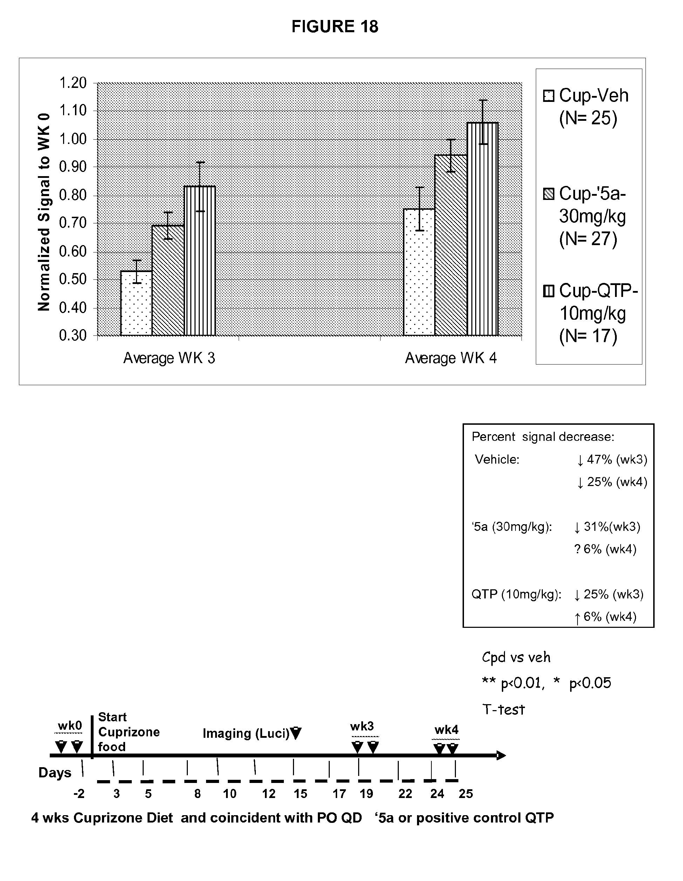

**Figure 18****:** '5a (30mg/kg) and QTP (10mg/kg) groups have significantly greater transgene activity compared to the vehicle control group at weeks 3 and 4. Imaging model data support that both QTP and '5a attenuate cuprizone-induced brain demyelination and myelin breakdown.

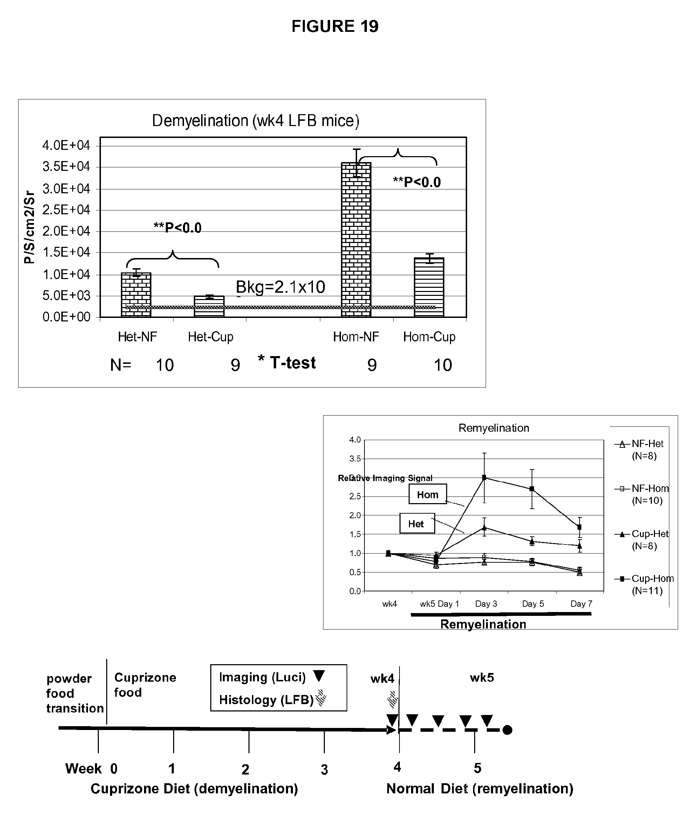

**Figure 19** shows cuprizone model imaging signal comparison between homozygous and heterozygous mice (B6C3H line 171). N3 generation heterozygous mice were interbred to produce homozygous mice for the MBP-luci allele. Homozygous mice showed a greater than 2-fold bioimaging signal window than heterozygous mice during cuprizone treatment. Two copies of the reporter gene in the homozygotes showed greater than two-fold signal decrease during the demyelination phase (e.g., after 4 weeks on cuprizone diet) and a two-fold signal increase during the remyelination phase (e.g., 1 week after removal of the cuprizone diet and return to normal chow).
   Although data have demonstrated that the heterozygous line 171 (B6C3H strain) works in the cuprizone model and can detect compound effects, the model could be further improved by breeding to homozygosity to increase bioimaging signal intensity. This also streamlines model production and decreases genotyping costs, since mouse colonies can be maintained as homozygotes. Overall, a larger imaging window can augment detection of compound effects in pharmacological compound profiling studies.

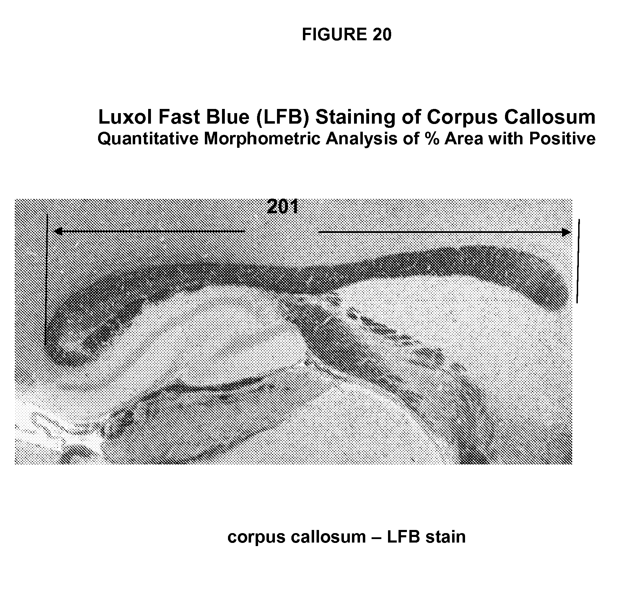

**Figure 20** Photomicrograph of the corpus callosum (dark longitudinal structure within brackets - 201) in a parasagittal tissue section from formalin fixed paraffin embedded mouse brain stained with LFB demonstrates the area of white matter evaluated for myelin status. This area was used for quantitative digital imaging analysis of Luxol Fast Blue (LFB) staining of the corpus callosum.

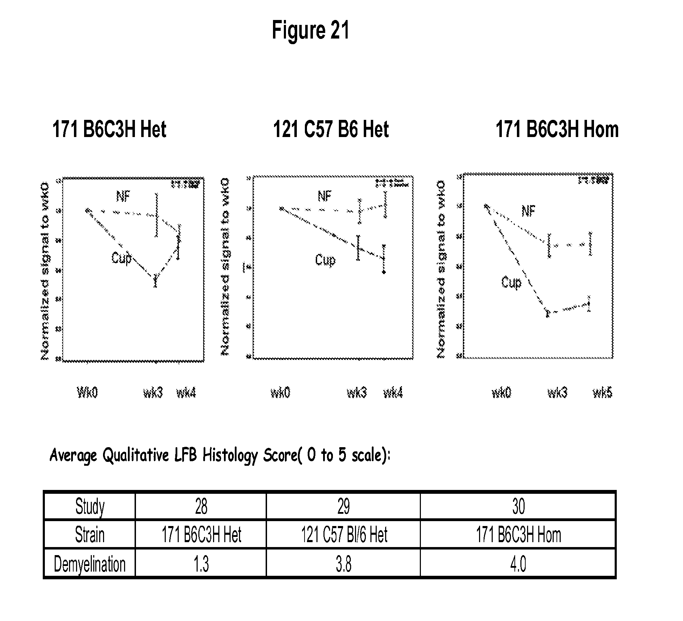

**Figure 21****:** Comparison of imaging window and histology window for three different MBP-luci lines (line 171 B6C3H heterozygous strain, line 121 C57BL/6 heterozygous strain and line 171 B6C3H homozygous strain). Mice were placed on a diet containing 0.2% cuprizone for 4 weeks or 5 weeks. Imaging data were normalized to week 0 baseline measurements. At the end of each study, mouse brains were harvested and seriai paraffin sections were stained for myelin with Luxol Fast Blue (LFB). Average qualitative LFB scores (0 to 5) are shown in the table. Line 171 B6C3H homozygous mice showed the largest imaging signal decrease and also demonstrated the most severe demyelination, as assessed by qualitative histology. Line 171 B6C3H heterozygous mice showed the smallest imaging window and also the least histological demyelination at week 4.

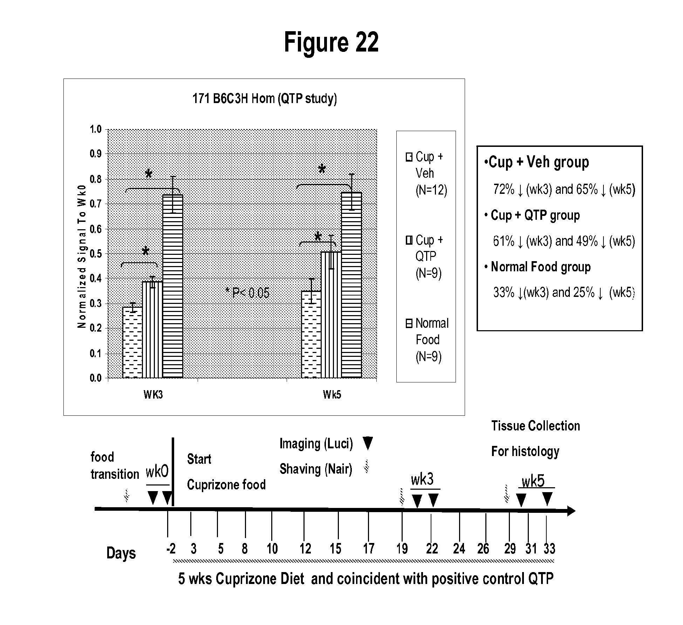

**Figure 22****:** Use of line 171 homozygous mice in the cuprizone model was further validated by demonstration of a treatment response to quetiapine (QTP). Mice were fed a cuprizone diet for 5 weeks and coincidently were orally dosed daily with QTP (10mg/kg). Mice were imaged at week 0 (baseline), week 3 and week 5. Data were normalized to week 0 baseline measurements. QTP (10mg/kg) caused significant increases in the imaging signal compared to the vehicle control, at both the week 3 and week 4 time points. Results are consistent with those obtained with line 171 heterozygous mice (Figures 17 and 18). Data indicate that line 171 homozygous mice can be used to assess the effect of compounds on maintaining myelin expression and integrity in the cuprizone model.

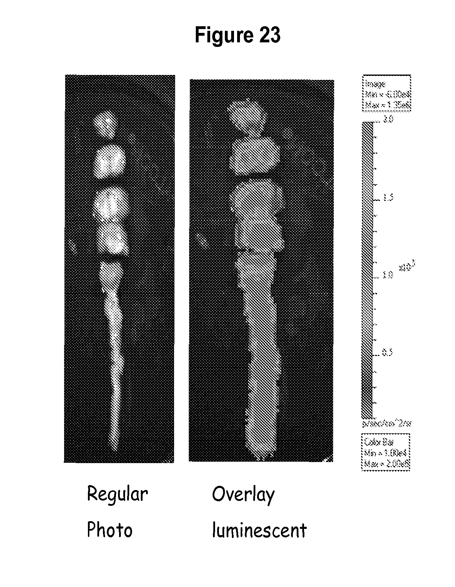

**Figure 23****:** *Ex vivo* spinal cord imaging from a line 121 mouse. Overlay of luminescence illustrates that transgene expression was localized to the white matter regions of the brain and spinal cord. This further supports the conclusions reached from the whole animal bioimaging experiments.

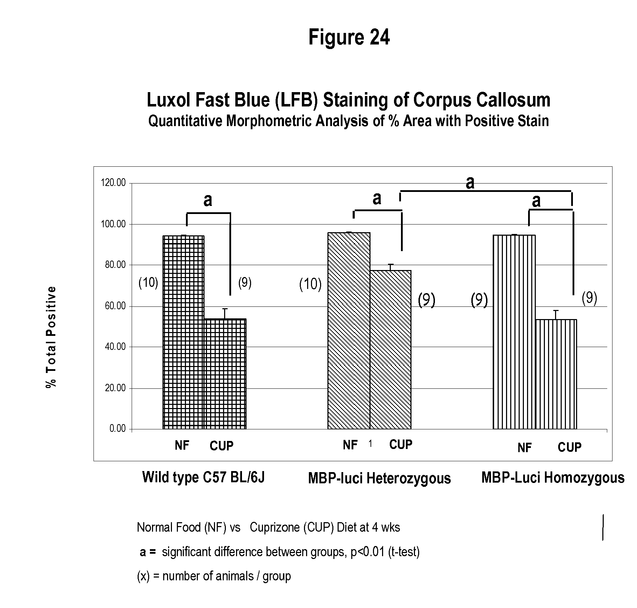

**Figure 24** Quantitative digital image analysis of Luxol Fast Blue (LFB) staining of the corpus callosum in C57BI/6 mice; wild type, line 171 heterozygous and line 171 homozygous mice. Quantification of percent area with positive LFB stain within the corpus callosum was calculated using the Aperio^{®} color deconvolution algorithm on manually outlined areas of the corpus callosum on scanned digital images of stained slides. One section was evaluated per animal. The number of animals per group varied between 9 and 10. Percent area positive for LFB stain was calculated per animal and for groups. Statistical significance between groups was evaluated by paired t-test. Both 171 homozygous mice and wild C57 BL/6 mice show severe demyelination (% positive staining is between 40 to 60%) after 4 weeks cuprizone feeding. In contrast, line 171 heterozygous mice show only mild demyelination (% positive staining is between 65 to 80%). Therefore, line 171 homozygous mice were identified as the preferred line for use in the cuprizone induced demyelination model.

### DESCRIPTION OF THE INVENTION

The following five criteria have been successfully applied sequentially to optimize selection for a bioimaging model:

Specific embodiments can be created from lines of mice transgenically manipulated with either the 5K or 10K vectors according to the following process. Results described below are from one such selection process that started with 35 transgenic lines. Figure 5 graphically summarizes this process.

From generated transgenic lines, one selects lines in which *in vivo* whole animal, e.g., mouse, imaging at the peak of postnatal myelination (e.g., 3-5 weeks G1 mice) shows CNS specific imaging. Six out of the selected 35 lines were advanced to the next selection stage.

Next lines in which *ex vivo* imaging confirms luciferase transgene expression mainly in the white matter region of brain were selected. Five of the 6 lines from step 1 were advanced to the next stage.

Then lines in which the luciferase image intensity highly correlated with changes in demyelination and remyelination induced in, in this example, a Cuprizone demyelination model was selected. Three of the 5 lines from step 2 were advanced to the next stage.

Next lines which showed clear histological demyelination in the above Cuprizone model were selected. Two of the 3 from step 3 were selected as preferred lines.

As a final proof of concept we selected one line, in which the efficacy of A003398711, a PPARδ selective agonist, was optimally detectable in the bioimaging model.

An exemplary line designated line 171 (B6C3 strain, heterozygous) was selected using the above five criteria and used as a preferred model.

Amore detailed description of this process is described in examples below.

### Definitions

As used herein unless indicated otherwise terms have meanings as generally used in the science parlance which may differ from colloquial common usage.

A gene should be interpreted broadly to include transcribed as well as non-transcribed regions.

Compound is interpreted broadly to include chemical compounds, e.g., organic chemical entities, biologic compounds, e.g., antibodies and antigen recognizing fragments and constructs, nucleic acids, e.g., RNAi, etc.

Transgenic mice that expressed firefly luciferase were generated. In these animals, the reporter gene, luciferase, was linked to an MBP promoter, thus driving expression of luciferase in cells of white matter (myelinated) region e.g., of the CNS when MBP expression was turned on. Systemic injection of the substrate luciferin (IV, IP, SC) generates a detectable and quantifiable light signal from a living mouse's head. By applying a cuprizone demyelination model to select MBP-luci lines and injecting these animals with luciferin repetitively, one can serially monitor and quantify demyelination and remyelination through noninvasive bio-luminescence imaging longitudinally, for example, over a two month period. This model successfully quantitatively monitored a cuprizone-induced demyelination and a PPARδ compound-induced remyelination.

Advances in detector technology have led to substantial improvement in sensitivity and image quality Photons are now detected by specialized charge coupled device (CCD) cameras that convert photons into electrons as photons strike silicon wafers. CCD cameras spatially encode the intensity of incident photons into electrical charge patterns which are then processed to generate an image. The noise is reduced by super-cooling the CCD camera and mounting the camera in a light-tight box. These cameras are generally controlled by a computer during image acquisition and analysis. Second-generation camera systems that are much smaller and therefore can be accommodated on laboratory bench tops made the technology feasible and practical for day-to-day experimentation. Xenogene Company has commercialized bioimaging technology.

Of the imaging modalities available, optical techniques based on bioluminescence or fluorescence have emerged as the most accessible and easily manipulated. Bioluminescent imaging (BLI) is characterized by remarkable sensitivity, as background luminescence (noise) from tissues is exceedingly low. To date, BLI has been successfully used to monitor biological processes such as cell movement, tumor progression, gene expression, and viral infection in a variety of animal models.

Firefly luciferase requires intracellular cofactors such as ATP for activity. This limited its use to cells that were genetically engineered to express the enzyme. As a result, many useful imaging applications, such as, monitoring distribution of circulating factors, detecting extracellular antigen expression, and labeling endogenous cells are not amenable to firefly luciferase imaging. An additional drawback of firefly luciferase is the lack of alternative substrates for detecting it in fixed cells and tissue samples. This has made it difficult to correlate *in vivo* imaging with microscopic analysis.

Sensitivity of detecting light emitted from internal organs depends on several factors, including the level of luciferase expression, the depth of labeled cells within the body (the distance that the photons must travel through tissue), and the sensitivity of the detection system.

The monitoring of expression of luciferase reporter expression cassettes using non-invasive whole animal imaging has been described (Contag, C. , U.S. Pat. No. 5,650,135, Jul. 22, 1997, Contag, P., et al, Nature Medicine 4(2):245-247, 1998; Contag, C., et al, OSA TOPS on Biomedical Optical Spectroscopy and Diagnostics 3:220-224, 1996; Contag, C. H., , Photochemistry and Photobiology 66(4):523-531, 1997; Contag, C. H., al, Molecular Microbiology 18(4):593-603, 1995). Such imaging typically uses at least one photo detector device element, for example, a charge-coupled device (CCD) camera.

### Control Elements of MBP gene

Myelin basic proteins (MBPs) are a family of polypeptides that are predominantly expressed in the nervous system where they play a major role in myelination. Expression of MBP, for example in differentiating oligodendrocytes is mainly regulated at the transcriptional level. In the Journal of Neuroscience, Farhadi *et al.* described a new regulatory combinatorial element that temporally controls expression of the MBP gene.

Farhadi *et al* showed that glia use different combinations of regulatory sequences to control expression of MBP at various stages during and after the onset of myelination.

Myelin basic protein (MBP) is required for normal myelin compaction and is implicated in both experimental and human demyelinating diseases, like MS.

In order to further advance understanding of myelin biology and test myelin enhancement compound in living animals. The present invention used the MBP promoter qualities and the most sensitive luciferase reporter technology to generate the present MBP-luci model. The model now permits sensitive *in vivo* measurements of myelin gene transcriptional responses in living animals.

### Construction of MBP-luci report cassettes

The 129SvEv BAC library (Cell & Molecular Technologies) was screened with a probe located in MBP promoter M3 region. The probe was 507 bps and was generated with primer pair (5'-actccttaccacacttcttgcagg-3' 5'-tctattgggtgatgtgtgccatc-3). (SEQ ID Nos. 1 and 2) MBP BAC was confirmed with the same probe through Southern analysis (7.6K fragment digested using EcoRI and/or 13.8K digested with BamHI).

"Long" MBP promoter containing M1 through M4 (10K) amplified by high fidelity PCR with primer set (MBP-L-SP2 5-gggggatccacctgggacgtagcttttgctg and MBP-AP1 5-ggggtttaaactccggaagctgctgtggg) (SEQ ID Nos. 3 and 4) was cloned into Invitrogen's xI-topo vector to produce an intermediate vector (Topo MBP10k vector). Then MBP 10k promoter (BamH1 and Pmel fragment) was inserted into pGL3 hygro neo vector (BgIII and Pmel sites). The final 10K vector was called pGL3-hygro-long MBP-luci (see e.g., Fig 4).

"Short" MBP promoter containing M1 to M3 (5k) amplified by high fidelity PCR with primer set (MBP-S-SP2 5-gggggatccatccctggatgcctcagaagag and MBP-AP1 5-ggggtttaaactccggaagctgctgtggg) (SEQ ID Nos. 5 and 6) was cloned into Invitrogen's p2.1-topo vector to produce an intermediate vector (Topo MBP5k vector). Then MBP 5k promoter (BamH1 and Pmel fragment) was inserted into pGL3 hygro neo vector (BgIII and Pmel sites). The final 5K vector was called pGL3-hygro-short MBP-luci. (see e.g., FIG 3).

DNA sequences from both pGL3-hygo-MBP plasmids confirmed M1, M2, M3 and M4 reading. In addition, transient transfection of these plasmids into 293T cells gave detectable luciferase activity.

### Animal Handling and Generation of Transgenic Mice

All animal work was performed in accordance with federal guidelines. Three different strains of mice (FVB, B6C3 and C57 BL/6) have been used. Imaging was performed under inhalation anesthesia with isoflurane (Baxter, Deerfield, IL); mice were observed until fully recovered.

Transgenic mice were generated as follows: Either pGL3-hygro-MBP10k-luci or pGL3-hygro-MBP5k-luci plasmid was digested with Not I and BamH1 enzymes. A fragment containing the MBP promoter, luciferase and polyadenylation signal was then gel purified. Transgenic mice were generated by standard pronuclear injection into FVB, B6C3 or C57BL/6 embryos. In brief, during pronuclear microinjection, the MBP-luci gene cassette DNA is introduced directly into the mouse egg just after fertilization. Using a fine needle, the DNA is injected into the large male pronucleus, which is derived from the sperm. The DNA tends to integrate as many tandem arranged copies at a random position in the genome, often after one or two cell divisions have occurred. Therefore, the resulting mouse is only partially transgenic. If the transgenic cells contribute to the germ line, then some transgenic eggs or sperm will be produced and the next generation of mice will be fully transgenic.

Transgenic founders and their Tg+ G1 offspring were identified by polymerase chain reaction (PCR) of tail biopsy DNA using primers specific for the firefly luciferase gene (PCR primers: 5'gaaatgtccgttcggttggcagaagc-3', and 5'ccaaaaccgtgatggaatggaacaaca-3') (SEQ ID Nos. 7 and 8)

Offspring of 25 positive founders were imaged using the *In vivo* Imaging System (IVIS 100; Xenogen, Alameda, CA), and six transgenic lines were identified with brain imaging signal (Two FVB lines and four B6C3HF1 lines). Since no brain imaging positive C57 BL/6 founder was generated in this exercise, one FVB line was backcrossed to C57BL/6 mice to achieve a C57 BL6 strain. B6C3 line 171 mice were subsequently propagated by intercrossing to achieve homozygous transgenic mating pairs. B6C3F 1 line 171 was also backcrossed to C57 albino line.

### TABLE 1

*In vivo* bioluminescence imaging was used to screen select MBP-luci lines. G1 mice were anesthetized with isoflurane, and a dose of 250mg/kg luciferin was injected through the tail vein or S.C. Eight minutes after the luciferin injection, mice were imaged. Six lines were identified with brain imaging signal (Fig 5, two FVB strain lines: 58 and 121 and four B6C3 strain line: 12, 23, 85 and 171). Except line 58 the other five lines showed ex *vivo* luciferase imaging signal at white matter region of brain.

Table 1 shows data from 35 transgenic DNA positive founder mice identified shortly after birth through tail biopsies PCR genotype. Fifteen DNA positive founder lines generated MBP-10k luci and twenty DNA positive founder lines generated MBP-5k luci. Throughout this application, the transgene and the transgenic mouse are abbreviated as MBP-luci.

**Table 1. MBP-lud has been generated as six different models to improve Bioimaging applications**

| **Model#** | **Strain** | **Het or Hom** | **Characteristic** |
|---|---|---|---|
| 538 | FVB | Line 121 Het | CNS and spinal cord expression |
| 557 | B6C3H | Line 171 Het | CNS expression only modulated by Cuprizone treatment |
| 551 | B6C3H | Line 171 Hom | CNS expression only modulated by Cuprizone treatment |
| 556 | C57 BL/6J | Line 121 Het | CNS and spinal cord expression |
| 565 | C57 Albino | Line 171 Hom | CNS expression only modutated by Cuprizone treatment ; Albino improves imaging |
| 595 | C57 Albino | Line 121 Het | CNS and spinal cord regression; Ablino improves imaging |

Figures 7 and 9 show MBP-luci bioluminescence correlated well with the CNS subregion and with age related myelination.

### Ex vivo imaging and luminometer assay

*Ex vivo* luciferase imaging of isolated organs was performed immediately after euthanasia of the animals by CO₂ 10 min after SC injection of luciferin (250 mg/kg). Dissected organs were placed on a black paper covered with plastic sheet and imaged by IVIS; strong bioluminescent signals remained detectable within 20 to 30 min after dissection. Image analysis and bioluminescent quantification was performed using Living Image software (Xenogen Corp.).

Tissue samples were placed in lysis buffer with inhibitors (Passive Lysis Buffer [Promega] and Complete Mini Protease Inhibitor Cocktail [Roche, Indianapolis, IN]). The tissues were homogenized using a tissue homogenizer. Tissues were further homogenized by brief sonication. Tissue homogenates were centrifuged and clarified lysates were used for luminometer assays. For the luminometer assays, Luciferase Assay Substrate (Luciferase Assay System, Promega) was prepared as indicated by the manufacturer. Tissue homogenates (20 µl) and substrate (100 µl) were mixed and measurements were taken in a luminometer. Background luminescence readings were obtained and the background readings were subtracted from the luminescent data. Protein concentrations were determined using the BCA Protein Assay Kit (Pierce, Rockford, IL) following the manufacturer's protocols. The luminescence for each of the protein lysates was calculated as arbitrary units of light per microgram of protein.

### Cuprizone induced demyelination and histology validation

Administration of Cuprizone to mice over a period of four weeks resulted in extensive demyelination of the corpus callosum. Cuprizone-induced demyelination is associated with significant microgliosis and macrophage recruitment (Bakker and Ludwin, J Neurol Sci 78: 125-37, 1987; Hiremath et al., J Neuroimmunol 92: 38-49, 1998; McMahon et al., J Neuroimmunol 130: 32-45, 2002), but does have minimal T-cell responses (Matsushima and Morell, Brain Pathol 11: 107-16, 2001). The consistent and predictable nature of the site of myelin injury in this model results in easily quantifiable change in corpus callosum myelination. These changes might result from the de-novo myelination by oligodendrocytes progenitor cells, however, prevention of terminal demyelination by immunomodulatory mechanisms (Pluchino et al., Nature 436: 266-71, 2005), might be a viable alternative explanation.

As outlined above, multiple strains of MBP-Luciferase Transgenic (MBP-luci Tg) mice were evaluated for *in vivo* assessment of cuprizone-induced demyelination/remyelination events. Expression of the myelin basic protein (MBP) promoter driven luciferase (luci) allowed *in vivo* bioimaging quantification of myelin in the brain of a transgenic (Tg) mammal expressing the MBP protein. The model, for example, can use wild type C57/BL6 mice fed 0.2% cuprizone in their diet. Previous models required terminal sacrifice at multiple time points for assessment of myelin after various compound treatments. Since multiple animals were required inter-animal variability was a factor requiring additional subjects (higher ns) to achieve significance.

The MBP 5k-luci line 171 (B6C3) mice showed prominent and significant demyelination in the corpus callosum of the brain as assessed by Luxol Fast Blue (LFB) histochemical staining on 0.2% cuprizone in the diet for four weeks. This demyelination was further correlated with a drop in the bioimaging *in vivo* luciferase signal.

The FVB strain, however, of MBP 10-Luci line 121 mice has not shown comparable demyelination. Additional studies were conducted in the FVB mouse in an attempt to identify a potential regimen of varying amounts of cuprizone in the diet and varying time periods of cuprizone treatment that might result in significant demyelination. The results showed only moderate amounts demyelination by LFB assessments in the corpus callosum. Accordingly, line 171 was preferentially developed.

### The impact of the different strain on cuprizone model:

Transgenic mice have frequently been created using the FVB/NJ (FVB) strain due to its high fecundity. FVB strain mice are also extensively used for transgenic bioimaging model due to their white relatively non-light absorbing fur color. Removal of hair, such as by shaving can also be used to reduce signal loss due to absorbance or scattering by hair or fur in FVB or other strains.

Because interstrain differences were observed for the cuprizone model, selection of strain may affect results. Selection of an optimal strain for a particular purpose is considered routine optimization dependent, for example, on selected assay and equipment. However, creating the transgenic mammal is not considered a limiting factor; rather the susceptibility of the particular strain and transgenic line to, e.g., a myelination affecting condition is used as a selection criterion for improving data quality. Depending on the myelination/demyelination event chosen, a choice of strain or genetic background may affect results. It is believed that specific demyelination models may work better in particular strains. Such choice of model and strain would be considered routine as part of assay development. Although cuprizone feeding is an excellent model in which to study demyelination and remyelination, there are strong genetic factors in this model apparently observed in strain differences.

### FVB strain:

The mice from the FVB strain were chosen in part due to their white fur color. FVB mice offer a system suitable for most transgenic experiments and subsequent genetic analyses. For example, the inbred FVB strain is characterized by vigorous reproductive performance and consistently large litters. This reduces cost and effort in producing large populations. Moreover, fertilized FVB eggs contain large and prominent pronuclei, which facilitate microinjection of DNA. In addition, the FVB strain has albino fur color and makes it a first choice for bioimaging. These features make the FVB strain advantageous to use for research with transgenic bioimaging models. However, other strains can be used when they exhibit desired characteristics.

FVB strain mice are very sensitive to 0.2% cuprizone in term of weight loss. Two to three times normal food/transgel supplement per week is required to avoid severe weight loss and toxicity. Furthermore the inventors' experience showed that FVB strain mice show minimal histological demyelination from various cuprizone feeding regimens.

Accordingly, the MBP 10k-luci line 121 (FVB strain) was backcrossed to C57 BI/6 to facilitate future validation and application.

### B6C3/Tac strain:

The B6C3 hybrid strain can be developed by intercrossing C57 BL/6Ntac female mice to C3H/HeNTac male mice from Taconic US's commercial colonies. It has black or agouti fur color. The B6C3 will be heterozygous at the loci where the C57BL/6 and the C3H differ, and homozygous at the loci where they are the same.

B6C3/Tac mice showed clear histological demyelination. Specifically demyelination in the bioimaging model line 171 MBP 5k-Luci homozygous mice was just mildly less extensive with mild variability as compared to wild type C57BL6. Demyelination in Line 171 MBP 5k-Luci heterozygous mice was considerably less severe, more localized and more variable as compared to C57BL6 & Line 171 MBP 5k-Luci homozygous mice. These results illustrate that the MBP-luci model can be useful in determining susceptibility of an individual, strain or species to a myelination affecting event. Furthermore, the MBP-luci construct does not lose usefulness even in black furred mammals.

### BALB/cJ strain:

Effect of cuprizone on cortical demyelination in BALB/cJ mice was also investigated. In these mice, cortical demyelination was only partial.

Moreover, cortical microglia accumulation was markedly increased in BALB/cJ mice, whereas microglia were absent in the cortex of C57BL/6 mice. Thus strain differences may be useful to support different research goals.

### C57 BL/6J Jax Strain:

C57BL/6 genetic background animals are suitable for many cuprizone model studies and have been used in several laboratories over the past 3 decades. When 8 week old C57BL/6 mice are fed 0.2% cuprizone in the diet, mature olidgodendroglia are specifically insulted (cannot fulfill the metabolic demand of support of vast amounts of myelin) and go through apoptosis. This event is closely followed by recruitment of microglia and phagocytosis of myelin. Studies of myelin gene expression, coordinated with morphological studies, indicate that even in the face of continued metabolic challenge, oligodendroglial progenitor cells proliferate and invade demyelinated areas. If the cuprizone challenge is terminated, an almost complete remyelination takes place within a matter of weeks. Intercellular communication between different cell types by soluble factors may be inferred. The method and model of the invention may there be useful for studying intercellular communication events, e.g., determining whether a putative factor is involved in recruitment for myelination, screening for compounds that facilitate recruitment, and screening for compounds inhibiting recruitment.

Furthermore, the reproducibility of the MBP-luci model indicates that it may permit testing of manipulations (e.g. available knockouts or transgenics on the common genetic background, or interfering RNA or pharmacological treatments) which may accelerate or repress the process of demyelination and or remyelination.

### Improvement of MBP-luci model

Although the line 171 (B6C3H strain, heterozygote) has been shown to work in myelination/demyelination studies, the model could be further improved by (1) Breeding to homozygocity to increase bioimaging signal intensity and reduce model production and genotype cost; (2) Breeding to an Albino strain such as the C57 strain to reduce imaging signal attenuation with white fur and to reduce skin reaction after multiple Nair shavings; (3) Breeding the line 121 to the C57 BL/6 strain to match the in-house developed CNS cuprizone model strain.

We have now demonstrated that line 171 homozygous showed an over 2-fold bioimaging window than line 171 heterozygous line (two copies of reporter gene cassette per cell). Other experiments also demonstrated albino C57 responded to cuprizone model.

### Imaging Systems and Data Analysis

Bioluminescence was measured noninvasively using the IVIS imaging system (Xenogen Corp., Alameda, CA). The images were taken 10 min after i.p. injection of luciferin (250 mg/kg-1; Xenogene Corp.) as a 60-s acquisition, binning 10, unless otherwise stated in the text. During image acquisition, mice were sedated continuously via inhalation of ∼2% isoflurane (Abbott Laboratories Ltd., Kent, United Kingdom).

### Imaging System description:

The IVIS^{®} Imaging System 100 (Xenogene) was used to collect the data proving this invention. Xenogen's sensitive IVIS^{®} Imaging System 100 Series offers an adjustable rectangular field of view of, for example, 10-25 cm, allowing 5 mice or 2 large rats to be imaged, as well as one standard microtiter plate. The system features a 25 mm (1.0 inch) square back-thinned, back-illuminated CCD (charged couple device) camera, which is cryogenically cooled to about -90 to -120°C, for example, -105°C via a closed cycle refrigeration system (without liquid nitrogen) to minimize electronic background and maximize sensitivity. The CCD camera is designed for high-efficiency photon detection, particularly in the red region of the spectrum. It can detect very small numbers of photons, as well as operate as a traditional camera; displaying images in that wide signal range is a function of Xenogen's Living Image^{®} software. There is a six-position filter wheel to isolate different bandwidths. This spectral information can reveal more about the depth and distribution of the source cells. The CCD is cooled and the electronic readout is optimized so that the data gathered to create the real-time *in vivo* images have extremely low noise.

### Light-Tight Imaging Chamber

An extremely light-tight, low background imaging chamber allows the IViS^{®} Imaging System 100 Series to be used in standard lab lighting environments. The sample shelf in the imaging chamber moves up and down to adjust the field of view. Researchers can view an entire animal, or focus on one portion for added detail. The shelf is heated to enhance the well-being of the anesthetized, e.g., mice or rats. The system includes animal handling features such as a heated sample shelf, gas anesthesia connections, and a full gas anesthesia option from Xenogen - the XGI-8 Gas Anesthesia System, shown on the website page. A larger imaging chamber could allow use of larger test subjects or a larger number of test subjects

### Preparation of Luciferin for In vivo Bioluminescent Assay:

The following materials were used in examples:
D-Luciferin Firefly potassium salt 1.0g /vial (e.g., Xenogen XR-1001 or Biosynth L-8220).
DPBS without Mg²⁺ and Ca²⁺.
Bottle top filter 0.2um.

The following procedure was used for imaging:
A stock solution of luciferin at 25 mg/ml in DPBS was prepared and filter sterilized through a 0.2um filter. 5 ml aliquots were store at -20°C. Injection dose was 10 ul/g of body weight. Each mouse was targeted to receive 250 mg luciferin/kg body weight (e.g. for 20g mouse, inject 200ul to deliver 2.0 mg of luciferin). Luciferin was injected SC, or IP, or IV several minutes before imaging. A luciferin kinetic study was optionally performed for each animal model to determine peak signal window.

### MBP-luci imaging method:

As described above, mice were injected with 250 mg/kg D-luciferin through SC, IP or IV. After 5 (intravenous) or 8 (intraperitoneal or SC) minutes, mice were imaged using the IVIS 100 (Xenogen) for 16 minutes (60 seconds imaging and 60 seconds interval for 8 pictures at bin size 8). To quantify bioluminescence, identical circular regions of interest were positioned to encircle each mouse head region, and the imaging signal was quantitated as average radiance (photons/s/cm2/steridian) using LIVINGIMAGE software (version 2.5, Xenogen). The head region of interest was kept constant in area and positioning within all experiments. Data were normalized to bioluminescence at the initiation of treatment for each animal.

### Statistical Analysis

For statistical analysis, EverStat V5 and Sigma Stat statistics software packages were used. The average of imaging in the group was taken as the mean, and SE for all groups were calculated.

When comparing two group means, a paired Wilcoxon test or unpaired Wilcoxon test was conducted. Two-tailed values of P < 0.05 were considered statistically significant.

### EXAMPLES

### Transgenic Mouse Generation

"Long" promoter is about 10 KB containing M1, M2, M3, M4 and "short" promoter is about 5KB containing M1, M2 and M3. These were cloned with a high fidelity PCR method from a mouse Bacterial Artificial Chromosome (BAC) containing a MBP gene. Then each promoter fragment was cloned into a vector, for example into the into the poly link site of a pGL3-hygro vector (Fig 1 and Fig 2).

The plasmids were restricted with Not I and BamH1 to release the MBP-luci transgenic expression cassettes (Fig 3) that were used to generate transgenic mice in the FVB/Tac and in B6C3/Tac strains using standard pronuciear microinjection techniques.

General strategies for generating transgenic (Tg) animals are well known in the art, for example as described in Pinkert, C. A. (ed.) 1994. Transgenic animal technology: A laboratory handbook. Academic Press, Inc., San Diego, Calif.; Monastersky G. M. and Robl, J. M. (ed.) (1995) Strategies in transgenic animal science. ASM Press. Washington D.C.).

### MBP-Luci Transgene Signal Correlated With Demyelination/Remyelination Events

MBP10K-luci transgenic line 121 (FVB strain) with observed white matter region luciferase expression was used in the Cuprizone model for validation experiments. As shown in the Fig 11, the first cuprizone study, was conducted with repeated luci imaging at wk1, 2 and 4 weeks (on 0.2% cuprizone diet) followed by a return to normal cuprizone absent food with imaging at 5, 6 and 7 weeks (RE wk1 through wk3). As shown in the figure, luciferase expression from this line 121 clearly correlated to cuprizone-induced demyelination and remyelination time courses. This is consistent with published endogenous MBP mRNA studies (Jurevics et al., Journal of Neurochemistry, 2002, 82, 126-136). FVB strain mice are but one strain and are known to be sensitive to 0.2% cuprizone as seen by weight loss. Routinely, two to three times normal food/transgel supplement per week is required to avoid severe weight loss and toxicity in this strain.

### Cuprizone model validation:

One well known and widely used demyelination/remyelination model is the Cuprizone model in the mouse. This model involves dietary consumption of cuprizone, a cropper chelator (typically about 0.2% w/w; biscyclohexanone oxaldihydrazone, CAS# 370-81-0, Sigma C9012) administered in powdered rodent lab chow for a period of, for example, four to six consecutive weeks (See, for example: Matsushima and Morell, 2001). Cuprizone has been shown to be selectively toxic to matured oligodendrocytes. Subsequent switch of the Cuprizone food to normal food creates an environment conducive to recovery, such that four to six weeks after ceasing Cuprizone feeding, the mice will exhibit extensive remyelination in the corpus callosum. Thus, the Cuprizone model provides a complete *in vivo* paradigm within which to study aspects of demyelination and remyelination (Figs 11 and 12).

As further proof, a MBP 5k-luci line 171 (B6C3 strain) was tested. This strain also showed a correlative imaging response to cuprizone-induced demyelination and remyelination events, as shown in the Fig 13. 7 Tg⁺ mice were treated with 0.2% cuprizone for 6 week and 3 Tg+ mice with normal food for 6 weeks. All 7 mice tolerated 0.2% cuprizone diet and had an average weight loss between 15 to 25%.

There was significant imaging signal drop with cuprizone treatment (demyelination). For example, there is 43% signal drop from wk 0 to wk 4 and 74% signal drop from wk 0 to wk 6.

### MBP-luci mice Cuprizone Model Histology Validation

For histology validation, an objective was to confirm, in the bioimaging model, that the response of the reporter gene during cuprizone treatment correlated with structurally detectable demyelination in the corpus callosum of Cuprizone-treated mice. These pathological conditions were visualized by Luxol Fast Blue (LFB) staining (see figures 20 and 24).

Specifically, for the MBP 10k-luci line 121 (FVB strain), with 0.2% cuprizone feeding, in initial trials, only minimal demyelination was detected through LFB staining. In order to generate clear histological demyelination for these FVB strain mice, various cuprizone feed regimens were followed to attempt to avoid severe weight loss. The 0.2%, 0.175% and 0.15% Cuprizone dose groups (6 week study) required 3-4 times normal food/transgel supplement per week to avoid severe weight loss and toxicity. Also, cuprizone concentration was further lowered with extended exposure time. Studies with cuprizone concentrations (0.14%, 0.12% and 0.1%) and treatment time (7 weeks and 9 weeks) were tested with up to once a week normal food/transgel supplement. However, all data showed that FVB strain mice (8 weeks old, weight 28.5 g ±3 g) had lesser histological demyelination from varied cuprizone feeding regimens. The FVB line was not selected as an especially preferred embodiment for preliminary development of this research model. Although FVB strain is good for imaging and sensitivity to cuprizone toxicity, weight loss might introduce confounding variables that could be easily avoided in these preliminary studies by using another strain.

In another strain, MBP 5k-luci line 171 (B6C3) mice (8 weeks old, weight 25 g ± 3 g) showed clear histological demyelination.

Mouse brain tissues had been collected at the end of six weeks 0.2% cuprizone treatment. All seven cuprizone treated mice had clear demyelination at the corpus callosum region and all three control mice showed normal myelination at the corpus callosum region. These data from the MBP 5K-luci line 171 provide further evidence that imaging signal tracks the cuprizone-induced demyelination phase.

Additional Quantitative LFB analysis (Fig 14) demonstrated that MBP- Luci B6C3 line 171 homozygous mice (8 weeks old, weight 21 g ± 3 g) showed more consistent and mildly more severe demyelination at 4 weeks compared to B6C3H line 171 heterozygous mice (8 weeks old, weight 25.5 g ± 3 g). The denser regions in the ovals show stained myelin.

Furthermore, C57BI/6 strain wild type male mice (8 weeks old, weight 20 g ± 3 g) fed with 0.2% cuprizone showed the most severe and consistent demyelination. C57 BL/6 strain has been served as positive control line for cuprizone model and PPARδ test compound effect described in preliminary studies.

### 1. MBP-luci mice confirm a PPARδ selective agonist tool compound's positive effect on CNS remyelination:

MBP-luciferase mice have been used to assess whether this *in vivo* bioimaging model can be used to detect a peroxisome proliferator-activated receptor δ (PPARδ) agonist tool compound ('571) effect on CNS remyelination.

The peroxisome proliferator-activated receptors (PPARs) belong to the nuclear receptor super-family that functions as transcription factors that regulate the expression of target genes. In contrast to other transcription factors, the activity of nuclear receptors can be modulated by binding to the corresponding ligands-small lipophilic molecules that easily penetrate biological membranes. Despite the complex cell signal pathway for the nuclear receptor family, there has been a long successful history to use nuclear receptors as drug targets. PPARs play essential roles in the regulation of cellular differentiation, development and metabolism. PPARs have three closely related isoforms encoded by separate genes identified thus far: commonly known as PPARa, PPARγ and PPARδ, also known as PPARβ; J. Berger and D. E. Miller, Annu. Rev. Med., 2002, 53, 409-435). Each receptor subtype has a signature DNA binding domain (DBD) and a ligand-binding domain (LBD), both being necessary for ligand activated gene expression. PPARs bind as heterodimers with a retinoid X receptor (RXR).

PPARδ appears to be significantly expressed in the CNS; however much of its function there still remains undetermined. Of singular interest however, is the discovery that PPARδ was expressed in rodent oiigodendrocytes, the major lipid producing cells of the CNS (J. Granneman, et al., J. Neurosci. Res., 1998, 51, 563-573). Moreover, it was also found that a PPARδ selective agonist was found to significantly increase oligodendroglial myelin gene expression and myelin sheath diameter in mouse cultures (I. Saluja et al., Glia, 2001, 33, 194-204). PPARδ knockout mice have smaller overall brain size and reduced levels of myelination in white matter (Mol cell Biology 200 20:5119). Additionally, PPARδ agonists exert protective effects in an experimental autoimmune encephalomyelitis (EAE) model of Multiple Sclerosis (Polak et al., J Neuroimmunology 2005 168:65-75).

Colleagues had previously demonstrated that selective PPARδ agonists play a functional role in neural tissue and stimulates oligodendrocyte progenitor cell differentiation. SAR117145, an orally bioavailable brain penetrable PPARδ selective agonist, stimulated rodent and human oligodendrocyte progenitor cells differentiation in vitro in a concentration dependent manner; an effect that could be blocked with a PPARδ selective antagonist.
In rat oligodendrocytes, increased expression of myelin basic protein was preceded by increased mRNA expression of the downstream PPAR target, Angptl4, and this upregulation was blocked with lentiviral shRNA knockdown of PPARδ. In a mouse cuprizone model of acute demyelination where mice were fed a diet of 2% cuprizone for 4 weeks, SAR117145, enhanced CNS remyelination, increased Angptl4 mRNA expression, and improved axonal conduction across the corpus callosum. CNS activation of Angptl4 was accompanied by increased expression in gastrocnemius muscle, suggesting that this could serve as a potential surrogate marker. These data demonstrate that PPARδ agonists can enhance CNS remyelination and improve axonal function and suggest their potential use in stimulating endogenous repair processes for the treatment of demyelinating disorders (US Patent application 20070149580, USE OF PEROXISOME PROLIFERATOR ACTIVATED RECEPTOR DELTA AGONISTS FOR THE TREATMENT OF MS AND OTHER DEMYELINATING DISEASES).

A PPARδ agonist tool compound ('517; Fig 15) was tested in B6C3H line 171 heterozygous mice. Eight week old mice were placed on a diet containing 0.2% Cuprizone for 4 weeks, then given normal diet for remyelination. Mice were then orally dosed twice daily with either vehicle (0.6% Carboxymethylcellulose sodium salt and 0.5% Tween 80) or 30mg/kg PPARδ agonist tool compound '517 for 8 days and imaged at the time points indicated in the graph. Data were normalized to week 0 baseline signals. There was a 30-100% relative luciferase signal increase in the tool compound '517 group (n=15) over vehicle group (n=13), which we believe is due to enhancement of oligodendrocyte progenitor cell differentiation.

The effect of '517 was further histologically confirmed by Luxol Fast Blue (LFB) staining in the same study (Figure 16). Parasagittal tissue sections from formalin fixed paraffin embedded brain were stained with LFB for qualitative assessment of myelin in the corpus callosum. The stained sections for each time point were scored and graded on a scale from 0 (complete myelination) to 5 (complete demyelination). Scoring system was as follows: 0 = normal myelin, no demyelination, 1 = minimal, localized demyelination, 2 = mild to moderate, localized demyelination, 3 = moderate, locally extensive demyelination, 4 = severe, locally extensive demyelination, 5 = severe, diffuse demyelination. Histological evaluation of LFB-stained brain sections from mice, after 4 weeks on cuprizone, confirmed moderate to severe demyelination of the corpus callosum in line 171 heterozygous mice (n=5). Treatment with '517 from week 4 to week 5 during remyelination phase resulted in a measurable increase in myelin as determined by LFB at the 7 week time point, tool compound '517 group (N=5) compared to vehicle control group (N=3). Despite small n-numbers, histological data support the *in vivo* luciferase bioimaging data, indicating increased remyelination in mice treated with the '517 compound when compared to vehicle controls.

A second PPARδ 517 study with line 171 heterozygous mice showed the similar results (data not shown, and also extended '517 treatment to three weeks). Consistent with the first study (Figure 15), the second study also suggests that '517accelerated oligodendrocyte progenitor cell differentiation at the early recovery phase during remyelination process (M Lindner and S Heine, et al, Neuropathology and Applied Neurobiology, 34, 105-114, 2008).

### 2. MBP-luci mice detect the protective effects of an ERβagonist, '5a or a positive control compound QTP on myelin expression.

The estrogen receptors (ERs) belong to the family of steroid nuclear receptors that act directly on the DNA through specific responsive elements and modulate gene expression. There are two subtypes of ERs, ERα and ERβ. ERα is highly expressed in the uterus, prostate, ovary, bone, breast and brain, whereas ERβ is present in the colon, prostate, ovary, bone marrow and brain. Selective targeting of ERβ is an attractive therapeutic approach to avoid ERα side effects. ER subtype-selective compounds have been identified.

For example, ERβ (not ERα) agonists have been shown to protect oligodendrocytes and neuroblastoma cell apoptosis *in vitro.* In addition, ERβ or ER-agonist ameliorates EAE and has neuroprotective effect (preservation of myelin and axons). Based on previous PPARδ agonist tool compound, '517, the MBP-luci line was used to profile an ERβ agonist, '5a in the cuprizone model. Furthermore, we included AstraZeneca's schizophrenia drug, Quetiapine (10 mg/kg, PO, qd) as a positive control based on its protective effect on cuprizone-induced demyelination (Schizophrenia Research, 2008 Dec 106, 182-91). Two independent studies were performed with B6CH line 171 heterozygous mice.

The first study (Figure 17) showed that the ERβ agonist, '5a and a positive control, Quetiapine (QTP), were protective during the period of demyelination in the cuprizone model. MBP-luc line 171 heterozygous B6C3H mice were fed a diet of cuprizone for 4 weeks and orally dosed with '5a (10mg/kg, N=12 or 30mg/kg N=16) or QTP (10mg/kg, N=14). Mice were imaged at week 0 (baseline), week 3 and week 4 data normalized to the week 0 baseline. Similar to previous study (Figure 15), the vehicle group resulted in a 49% (week 3) and 45% (week 4) bioimaging signal reduction. The QTP group showed significant increases in imaging signal vs. vehicle control for both week 3 and week 4 time points at 10mg/kg. Results for the QTP treatment group are consistent with data published by Zhang et al (Schizophrenia Research, 2008, 106:182-91). Compound '5a at 30mg/kg showed significant increases over vehicle at week 4, but showed no significant difference over vehicle at 10mg/kg at weeks 3 or 4. These results suggest that the MBP-luci imaging model can be used to assess dose dependent protective effects in the cuprizone model.

A further study (Figure 18) was designed to confirm the first study result (Figure 17) but with a larger cohort for the vehicle and the '5a compound 30mg/kg treatment groups. In agreement with the first study results, treating mice with QTP at 10 mg/kg (N=17) produced a statistically significant inhibition of cuprizone-induced signal decrease when compared to vehicle treated controls (N=25) at week 3 (25% vs 47% reduction, p=0.028) and at week 4 (6 % increase vs 25% reduction). Furthermore, '5a at 30mg/kg (N=27) results in significantly greater transgene activity compared to the vehicle control group at week 3 (31% vs 47% reduction, p=0.0079) and week 4 (6% reduction vs 25% reduction, p=0.0015).

These two studies demonstrated that '5a at 30mg/kg significantly prevented the cuprizone diet induced reduction in the bioimaging signal in the CNS although not to the same extent as the positive control QTP at 10mg/kg (under the conditions used in these experiments). Since previous studies have shown a direct relationship between the extent of CNS myelination and the MBP-luci bioimaging signal, these current results suggest that both '5a and QTP prevented demyelination in the CNS during cuprizone diet administration. Imaging model data support that both QTP and '5a attenuate the cuprizone-induced brain demyelination and myelin breakdown.

### 3. Comparison of the MBP-luci lines in the cuprizone model

Six transgenic lines on various strain backgrounds have been generated for distinct bioimaging applications.

B6C3H line 171 homozygous mice and heterozygous imaging signal in the cuprizone model (figure 19) was compared. Two copies of the reporter gene in the homozygotes showed greater than two-fold signal decrease during the demyelination phase and a two-fold signal increase during the remyelination phase. Although it is demonstrated that the heterozygous line 171 (B6C3H strain) works in cuprizone model and can detect compound effects, it is anticipated that the model could be further improved by breeding to homozygosity to increase bioimaging signal intensity. This would also streamline model production and decrease genotyping costs, since mouse colonies can be maintained as homozygotes. Moreover, the larger the imaging window, the more sensitive the model is towards detecting compound induced changes.

Figure 20 shows comparison of histological LFB data from three different lines in the cuprizone model. Quantitative LFB data confirmed the bioimaging results with line 171 B6C3H that homozygous mice exhibited the most severe cuprizone induced demyelination; similar in severity to that seen in wild type C57 BL/6 mouse strain typically used. Line 171 heterozygous mice line showed less severe demyelination while these Line 171 heterozygous mice showed the least amount of cuprizone induced demyelination.

In Figure 21, demonstrates that MBP-luci line with the largest bioimaging signal reduction also had the greatest demyelination, as assessed histologically. Three different MBP-luci lines (line 171 B6C3H het strain, line 121 C57BL/6 heterozygous strain and line 171B6C3H homozygous strain) were compared by bioimaging and Luxol Fast Blue (LFB; myelin stain) histology. Mice were placed on a diet containing 0.2% cuprizone for 4 or 5 weeks. Imaging data were normalized to week 0 baseline measurements. At the end of each study, mouse brains were harvested and serial paraffin sections were stained for myelin with LFB. Average qualitative LFB score (0 to 5) was shown in the chart of figure 21. Line 171 B6C3H homozygous mice showed the largest imaging signal decrease and also demonstrated the most severe demyelination as assessed by qualitative histological assessment. Line 171 B6C3H heterozygous mice showed the smallest imaging window and also the least histological demyelination at week 4. Line 171 B6C3H homozygous mice had the largest bioimaging signal reduction during cuprizone food feeding in the reference to their base line imaging before cuprizone food feeding. For example, after three weeks on the cuprizone diet, 171 B6C3H homozygous mice had 72% signal reduction (week 3 reference to week 0, p<0.05), while line 171 B6C3H heterozygous mice had 45% bioimaging signal reduction (reference to week 0, p<0.05). Line 121 C57 BL/6 heterozygous mice had the smallest reduction in luciferase signal (33% reduction at week 3 over week 0, p<0.05).

The sensitivity and responsiveness of MBP-luci model was further confirmed by treatment of 171 B6C3H homozygous mice with QTP (10 mg/kg) shown in Figure 22. Consistent with results using 171 B6C3H heterozygous mice (Figures 17 and 18) that QTP (10 mg/kg) significantly prevented bioimaging signal reduction. Based on these results, Line 171 B6C3H homozygous mice were identified as the optimal line for the cuprizone induced demyelination bioimaging model.

### 4. Additional Application of MBP-luci model

MBP-luci mice have both brain and spinal cord luciferase expression. As shown in Figure 23, luminescence signal was primarily from the white matter region of brain and spinal cord. Besides luciferase imaging from brain that has been successfully demonstrated for cuprizone model applications, luciferase imaging signal from spinal cord could be used in the experimental allergic Encephalitis (EAE) model of MS or applied as a spinal cord model.

## Claims

1. A method of monitoring demyelination or remyelination in a living rodent, said method comprising:
transgenically modifying progenitor cells to produce a rodent that expresses a luciferase gene controlled by a myelin basic protein (MBP) promoter, wherein said MBP promoter contains modules M1 through M4, or contains modules M1 through M3; monitoring bioluminescence from said rodent; and correlating said light output to specific portions of the body of said rodent; and further comprising:
repeating, in the same rodent, said monitoring at a time discrete from said initial monitoring; and comparing outputs of said monitoring events to observe change in myelination in the same rodent.

2. The method of claim 1, wherein said rodent is a mouse.

3. The method of claim 1 or 2, further comprising:
imaging signal normalization through demyelination or remyelination intervals, wherein said imaging through intervals is effective to detect changes in the level of MBP transcript in said rodent over said intervals.

4. A transgenic rodent comprising a luciferase gene driven through a myelin basic protein (MBP) promoter, wherein said MBP promoter contains modules M1 through M4, or contains modules M1 through M3.

5. A transgenic rodent expressing a luciferase gene, said luciferase gene being under control of a myelin basic protein (MBP) promoter, wherein said MBP promoter contains modules M1 through M4, or contains modules M1 through M3.

6. The transgenic rodent of claim 4 or 5, wherein said rodent is a mouse.

7. The method of claim 1, wherein said rodent has white hair or diminished hair, and wherein hair of said rodent preferably absorbs less light within a wavelength range of 530-640 nm than hair of a C57/B6 mouse.

8. A method for developing a bioimaging mouse strain comprising:
(1) selecting a line in which in vivo whole mouse imaging at a peak of post natal myelination shows central nervous system (CNS) specific imaging;
(2) selecting a line in which ex vivo imaging confirms luciferase transgene expression mainly in the white matter region of brain or spinal cord;
(3) selecting a line in which the luciferase image intensity is highly correlated with changes in demyelination and remyelination induced in a demyelination model; and
(4) selecting a line which shows clear histological demyelination as a model.

9. The method of claim 8, wherein said peak of post natal myelination is approximately 3-5 weeks G1 mice.

10. The method of claim 8, wherein said model is a cuprizone demyelination model.

11. The method of claim 1, wherein said method monitors the effect of a compound on at least one demyelination or remyelination event.

12. The method of claim 1, wherein said method monitors the effect of a gene event.

13. An improved method for monitoring myelination, the improvement comprising imaging a transgenic rodent expressing MBP-luciferase according to claim 5.

14. A method that reduces variability in myelination studies, said method comprising monitoring MBP-controlled luciferase in a transgenic rodent according to claim 5 at at least two discrete timepoints.

15. The method of claim 1, wherein a specific portion of the body of said rodent is selected from the group consisting of peripheral nervous system and central nervous system.

## Patentansprüche

1. Ein Verfahren zum Überwachen von Demyelinisierung oder Remyelinisierung in einem lebenden Nagetier, wobei besagtes Verfahren umfasst:
transgenes Modifizieren von Progenitorzellen, um ein Nagetier zu produzieren, das ein Luciferase-Gen, welches durch einen Myelin-Basisches Protein (MBP)-Promotor kontrolliert wird, exprimiert, wobei besagter MBP-Promotor Module M1 bis M4 enthält, oder Module M1 bis M3 enthält; Überwachen von Biolumineszenz von besagtem Nagetier; und Korrelieren besagten Licht-Outputs mit spezifischen Abschnitten des Körpers besagten Nagetiers;
und ferner umfasst:
Wiederholen besagten Überwachens in demselben Nagetier zu einem Zeitpunkt, welcher unterschiedlich ist von besagtem initialen Überwachen; und Vergleichen der Outputs besagter Überwachungsereignisse, um eine Änderung in der Myelinisierung in demselben Nagetier zu beobachten.

2. Das Verfahren gemäß Anspruch 1, wobei besagtes Nagetier eine Maus ist.

3. Das Verfahren gemäß Anspruch 1 oder 2, ferner umfassend:
Normalisierung des Bildgebungssignals durch Demyelinisierungs- oder Remyelinisierungsintervalle, wobei besagte Bildgebung durch Intervalle effektiv ist, um Änderungen im Level des MBP-Transkripts in besagtem Nagetier über besagte Intervalle zu detektieren.

4. Ein transgenes Nagetier, umfassend ein Luciferase-Gen, welches durch einen Myelin-Basisches Protein (MBP)-Promotor angetrieben wird, wobei besagter MBP-Promotor Module M1 bis M4 enthält, oder Module M1 bis M3 enthält.

5. Ein transgenes Nagetier, exprimierend ein Luciferase-Gen, wobei besagtes Luciferase-Gen unter der Kontrolle eines Myelin-Basisches Protein (MBP)-Promotors ist, wobei besagter MBP-Promotor Module M1 bis M4 enthält, oder Module M1 bis M3 enthält.

6. Das transgene Nagetier gemäß Anspruch 4 oder 5, wobei besagtes Nagetier eine Maus ist.

7. Das Verfahren gemäß Anspruch 1, wobei besagtes Nagetier weißes Haar oder vermindertes Haar hat, und wobei Haar besagten Nagetiers vorzugsweise weniger Licht innerhalb eines Wellenlängenbereichs von 530-640 nm absorbiert als Haar einer C57/B6 Maus.

8. Ein Verfahren zur Entwicklung eines Mäusestamms zur Bio-Bildgebung, umfassend:
(1) Auswählen einer Linie, in welcher in vivo-Bildgebung der gesamten Maus an einem Höchstwert postnataler Myelinisierung eine spezifische Bildgebung des zentralen Nervensystems (ZNS) zeigt;
(2) Auswählen einer Linie, in welcher ex vivo-Bildgebung die Luciferase-Transgenexpression hauptsächlich in der Region der weißen Substanz von Gehirn oder Rückenmark bestätigt;
(3) Auswählen einer Linie, in welcher die Luciferase-Bildgebungsintensität stark mit Änderungen in Demyelinisierung und Remyelinisierung, induziert in einem Demyelinisierungsmodell, korreliert; und
(4) Auswählen einer Linie, welche klare histologische Demyelinisierung als ein Modell zeigt.

9. Das Verfahren gemäß Anspruch 8, wobei besagter Höchstwert postnataler Myelinisierung der von ungefähr 3-5 wöchigen G1-Mäusen ist.

10. Das Verfahren gemäß Anspruch 8, wobei besagtes Modell ein Cuprizon-Demyelinisierungsmodell ist.

11. Das Verfahren gemäß Anspruch 1, wobei besagtes Verfahren den Effekt einer Verbindung auf mindestens ein Demyelinisierungs- oder Remyelinisierungsereignis überwacht.

12. Das Verfahren gemäß Anspruch 1, wobei besagtes Verfahren den Effekt eines Genereignisses überwacht.

13. Ein verbessertes Verfahren zum Überwachen von Myelinisierung, wobei die Verbesserung Bildgebung eines transgenen Nagetiers, welches MBP-Luciferase gemäß Anspruch 5 exprimiert, umfasst.

14. Ein Verfahren, das die Variabilität in Myelinisierungsstudien reduziert, wobei besagtes Verfahren das Überwachen von MBP-kontrollierter Luciferase in einem transgenen Nagetier gemäß Anspruch 5 zu mindestens zwei unterschiedlichen Zeitpunkten umfasst.

15. Das Verfahren gemäß Anspruch 1, wobei ein spezifischer Abschnitt des Körpers besagten Nagetiers aus der Gruppe bestehend aus peripherem Nervensystem und zentralem Nervensystem ausgewählt ist.

## Revendications

1. Procédé pour le suivi de la démyélinisation ou de la remyélinisation, chez un rongeur vivant, ledit procédé comprenant:
la modification par voie transgénique de cellules progénitrices pour produire un rongeur qui exprime un gène de luciférase régulé par un promoteur de la protéine basique de la myéline (MBP), tandis que ledit promoteur de MBP contient les modules M1 à M4, ou contient les modules M1 à M3; le suivi de la bioluminescence à partir dudit rongeur, et la corrélation de ladite sortie de lumière avec des portions spécifiques du corps dudit rongeur; et comprenant en outre:
la répétition chez le même rongeur, dudit suivi à un temps discret à partir dudit suivi initial, et la comparaison des sorties desdits événements de suivi pour l'observation d'une modification dans la myélinisation chez le même rongeur.

2. Procédé selon la revendication 1, dans lequel ledit rongeur est une souris.

3. Procédé selon la revendication 1 ou 2, comprenant en outre:
la normalisation du signal d'imagerie sur des intervalles de démyélinisation ou de remyélinisation, tandis que ladite imagerie par intervalles est efficace pour la détection de modifications dans le niveau de transcrit de MBP chez ledit rongeur au cours desdits intervalles.

4. Rongeur transgénique comprenant un gène de luciférase commandé par un promoteur de la protéine basique de la myéline (MBP), tandis que ledit promoteur de MBP contient les modules M1 à M4, ou contient les modules M1 à M3.

5. Rongeur transgénique exprimant un gène de luciférase, ledit gène de luciférase étant sous le contrôle d'un promoteur de la protéine basique de la myéline (MBP), tandis que ledit promoteur de MBP contient les modules M1 à M4, ou contient les modules M1 à M3.

6. Rongeur transgénique selon la revendication 4 ou 5, dans lequel ledit rongeur est une souris.

7. Procédé selon la revendication 1, dans lequel ledit rongeur a un pelage blanc ou un pelage réduit, et dans lequel le pelage dudit rongeur absorbe de préférence moins de lumière dans un intervalle de longueurs d'onde de 530-640 nm que le pelage d'une souris C57/B6.

8. Procédé pour développer une souche de souris pour bio-imagerie, comprenant:
(1) la sélection d'une lignée dans laquelle l'imagerie d'une souris entière in vivo à un pic de myélinisation post-natale présente une imagerie spécifique du système nerveux central (SNC);
(2) la sélection d'une lignée dans laquelle l'imagerie ex vivo confirme l'expression d'un transgène de luciférase principalement dans la région de la matière blanche du cerveau ou de la moelle épinière;
(3) la sélection d'une lignée dans laquelle l'intensité de l'image de la luciférase est fortement corrélée avec des modifications dans la démyélinisation ou la remyélinisation induites dans un modèle de démyélinisation; et
(4) la sélection d'une lignée qui présente une nette démyélinisation histologique, à titre de modèle.

9. Procédé selon la revendication 8, dans lequel ledit pic de myélinisation post-natale est approximativement des souris G1 de 3-5 semaines.

10. Procédé selon la revendication 8, dans lequel ledit modèle est un modèle de démyélinisation à la cuprizone.

11. Procédé selon la revendication 1, dans lequel ledit procédé procure le suivi de l'effet d'un composé sur au moins un événement de démyélinisation ou de remyélinisation.

12. Procédé selon la revendication 1, dans lequel ledit procédé procure le suivi de l'effet d'un événement génique.

13. Procédé perfectionné pour le suivi de la myélinisation, le perfectionnement comprenant l'imagerie d'un rongeur transgénique exprimant la luciférase de MBP selon la revendication 5.

14. Procédé qui réduit la variabilité dans les études de myélinisation, ledit procédé comprenant le suivi de la luciférase commandée par la MBP chez un rongeur transgénique selon la revendication 5 à au moins deux points de temps discrets.

15. Procédé selon la revendication 1, dans lequel une portion spécifique du corps dudit rongeur est choisie dans le groupe consistant en le système nerveux périphérique et le système nerveux central.
